# EUROPEAN PATENT APPLICATION

(11) **EP 4 729 605 A1**
(43) Date of publication of application: **22.04.2026**
(21) Application number: 24823302.5
(22) Date of filing: 06.06.2024
(51) Int. Cl.: C12N 1/21, C12P 7/04, C12N 15/53

(54) **CROTYL ALCOHOL-GENERATING BACTERIUM TRANSFORMANT OF GENUS HYDROGENOPHILUS**

(30) Priority: 15.06.2023 JP 2023098824
(71) Applicant: Utilization of Carbon Dioxide Institute Co.,Ltd., Tokyo 135-0091 (JP); ENEOS Materials Corporation, Tokyo 105-7109 (JP)
(72) Inventor: YUKAWA Hideaki, Tokyo 135-0091 (JP); HATORI Shin, Tokyo 105-7109 (JP); ATSUMURA Masatoshi, Tokyo 105-7109 (JP)
(74) Representative: Vossius & Partner Patentanwälte Rechtsanwälte mbB
(86) International application number: PCT/JP2024/020710
(87) International publication number: WO 2024/257686

(57) **Abstract**

A transformant obtained by introducing into a *Hydrogenophilus* bacterium any one of the following genes (1) to (5) is capable of efficiently producing crotyl alcohol utilizing only carbon dioxide as a carbon source.
(1) A gene encoding an enzyme having crotonaldehyde dehydrogenase activity and a gene encoding an enzyme having crotyl alcohol dehydrogenase activity;
(2) A gene encoding a multifunctional enzyme having both crotonaldehyde dehydrogenase activity and crotyl alcohol dehydrogenase activity;
(3) A gene encoding an enzyme having crotonaldehyde dehydrogenase activity and a gene encoding a multifunctional enzyme having both crotonaldehyde dehydrogenase activity and crotyl alcohol dehydrogenase activity;
(4) A gene encoding an enzyme having crotyl alcohol dehydrogenase activity and a gene encoding a multifunctional enzyme having both crotonaldehyde dehydrogenase activity and crotyl alcohol dehydrogenase activity;
(5) A gene encoding an enzyme having crotonaldehyde dehydrogenase activity, a gene encoding an enzyme having crotyl alcohol dehydrogenase activity, and a gene encoding a multifunctional enzyme having both crotonaldehyde dehydrogenase activity and crotyl alcohol dehydrogenase activity.

## Description

### Technical Field

The present invention relates to a *Hydrogenophilus* bacterium transformant having ability to produce crotyl alcohol, and to a method for producing crotyl alcohol using the same.

### Background Art

The Paris Agreement, which was adopted in 2015, provides that global emissions of greenhouse gases should be promptly reduced. Under the Agreement, Japan set the goal of reducing her emission of greenhouse gases such as carbon dioxide and methane by 46% compared with year 2013 levels, by the year 2030.

Worldwide, the majority of the production of chemicals depends on petroleum resources, exacerbating the problem of increased greenhouse gas emissions. Accordingly, departure from petroleum dependency is a desirable strategy for the production of chemicals, and research and development of biorefineries that produce green chemicals from biomass is being earnestly carried out in various countries. However, the saccharification of biomass for use as raw materials of microbial fermentation necessitates complex processes, besides being costly. Using biomass that could serve as food or feed for chemical production disturbs the stable supply of food and feed. Furthermore, the large-scale consumption of biomass for chemical manufacturing raises concerns that it actually leads to environmental destruction.

As part of research geared towards departure from petroleum dependency, gases such as carbon dioxide, methane, and carbon monoxide have attracted attention as more sustainable carbon sources, and techniques for producing valuable chemicals and biofuels using microorganisms that utilize these gases are the subject of interest. In particular, carbon fixation and efficient utilization of carbon dioxide, a significant contributor to global warming, is highly anticipated.

Butadiene is used as a raw material for synthetic rubbers such as butadiene rubber, styrene-butadiene rubber, acrylonitrile-butadiene rubber, and chloroprene rubber, as well as for synthetic resins such as acrylonitrile-butadiene-styrene (ABS) resin and methyl methacrylate-butadiene-styrene (MBS) resin. It is also utilized as a precursor for various other chemical products, including adiponitrile, 1,4-butanediol, cyclododecatriene, chloroprene, and sulfolane.

Butadiene is mainly produced by catalytic cracking wherein naphtha and steam are heated at high temperatures 900°C or more, which also generates other olefins and aromatic hydrocarbons. In recent years, environmentally conscious methods that use bioethanol as a raw material have been employed. However, in these methods, the conversion of ethanol into acetaldehyde and the conversion of the ethanol-acetaldehyde mixture into butadiene are carried out by chemical reactions, and thus do not sufficiently reduce environmental load.

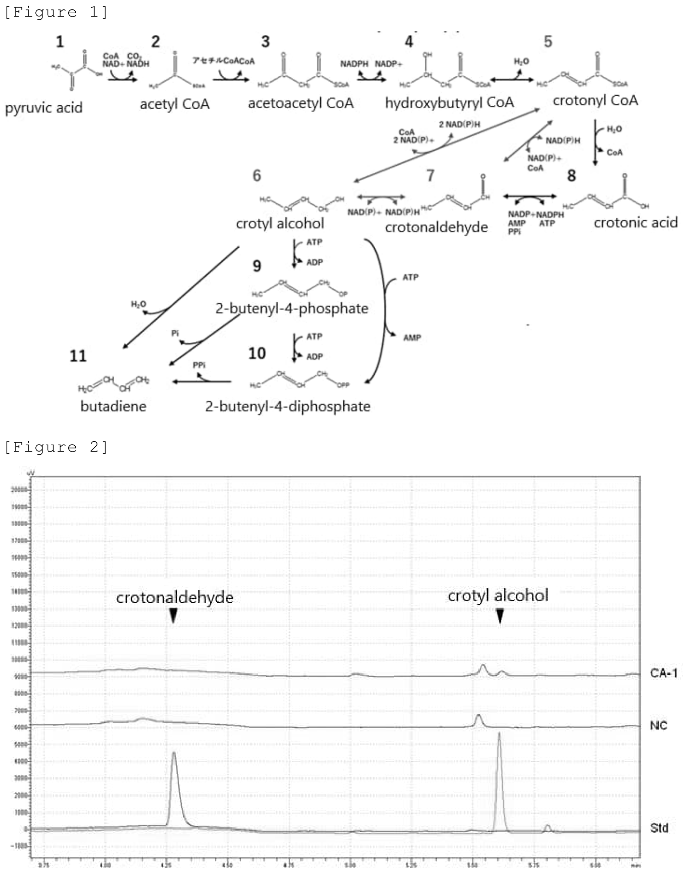

Accordingly, microbial production of butadiene has been tried. Many microorganisms possess most of the metabolic pathway from pyruvate to butadiene via crotonyl-CoA and crotyl alcohol (also known as crotonyl alcohol), as shown in Figure 1. However, no microorganism capable of producing butadiene has been identified. Therefore, various methods that produce butadiene by culturing a transformant obtained by introducing one or more exogenous genes encoding enzymes involved in this metabolic pathway are proposed.

For example, Patent Literature 1 discloses a method for producing butadiene that comprises a step of generating crotyl alcohol by culturing a *Escherichia coli* transformant, and a subsequent step of chemically converting the crotyl alcohol into butadiene (Claim 1). The transformant used in this method is engineered by introducing the following exogenous genes (A) and (B) encoding enzymes that catalyze the conversion from crotonyl-CoA to crotyl alcohol:
(A) One of the following (1) to (3):
   (1) An aldehyde-forming crotonyl-CoA reductase (Compound 5 to 7 in Figure 1) and an alcohol-forming crotonaldehyde reductase (Compound 7 to 6 in Figure 1)
   (2) An alcohol-forming crotonyl-CoA reductase (Compound 5 to 6 in Figure 1)
   (3) A crotonyl-CoA hydrolase (Compound 5 to 8 in Figure 1), crotonyl-CoA synthetase (Compound 5 to 8 in Figure 1), or crotonyl-CoA transferase (Compound 5 to 8 in Figure 1), together with a crotonic acid reductase (Compound 8 to 7 in Figure 1) and an alcohol-forming crotonaldehyde reductase (Compound 7 to 6 in Figure 1).
(B) Acetyl-CoA:acetyl-CoA acyltransferase (Compound 2 to 3 in Figure 1), acetoacetyl-CoA reductase (Compound 3 to 4 in Figure 1), and 3-hydroxybutyryl-CoA dehydratase (Compound 4 to 5 in Figure 1)

However, the method described in Patent Literature 1 uses glucose or starch derived from biomass as a carbon source (paragraph 0087). As noted above, the use of biomass involves complex processes to convert biomass into sugars, which increases production costs. Moreover, the industrial use of biomass actually causes environmental load.

Patent Literature 2 discloses a method for producing butadiene by bringing a transformant in contact with a carbon source in a culture medium (Claims 1 and 2), the transformant being prepared by introducing following exogenous gene (A) or (B) into a bacterium belonging to the genera *Burkholderia, Propionibacterium, Propionispira, Clostridium, Bacillus, Escherichia, Pelobacter,* or *Lactobacillus.*
(A) A crotonyl-CoA reductase (Compound 5 to 7 in Figure 1) and a linalool dehydratase, which is a type of crotonyl alcohol dehydratase (Compound 6 to 11 in Figure 1)
(B) A crotonaldehyde dehydrogenase (Compound 5 to 7 in Figure 1), a crotonyl alcohol dehydrogenase (Compound 7 to 6 in Figure 1), and a linalool dehydratase, which is a type of crotonyl alcohol dehydratase (Compound 6 to 11 in Figure 1).

However, the method described in Patent Literature 2 produces butadiene by cultivating the above microorganisms using sugars or similar compounds as carbon sources (e.g., paragraph 0088), and has disadvantages accompanied by use of biomass.

A method for producing crotyl alcohol, a precursor of butadiene, using microorganisms has also been proposed.

For example, Patent Literature 3 discloses a technique for producing crotyl alcohol from one or more C1 compounds selected from methane, methanol, methylamine, formic acid, formaldehyde, and formamide with use of a transformant (Claim 11 and paragraph 0001). The transformant is prepared by introducing at least one gene selected from a gene encoding acetoacetyl-CoA thiolase (Compound 2 to 3 in Figure 1), a gene encoding 3-hydroxybutyryl-CoA dehydrogenase (Compound 3 to 4 in Figure 1), a gene encoding 3-hydroxybutyryl-CoA dehydratase (Compound 4 to 5 in Figure 1), a gene encoding crotonyl-CoA hydrolase (Compound 5 to 8 in Figure 1), a gene encoding crotonic acid reductase Compound 8 to 7 in Figure 1), and a gene encoding alcohol-forming crotonaldehyde reductase (Compound 7 to 6 in Figure 1) as well as genes encoding enzymes that convert methanol and/or formic acid into formaldehyde and genes encoding enzymes that confer formaldehyde fixation capability, into a host, a methylotroph such as methylotrophic yeast.

However, C1 compounds such as methane are primarily synthesized with use of natural gas, coal, oil shale, and the like as raw materials. Therefore, the method described in Patent Literature 3 cannot be considered effective in reducing greenhouse gas emissions.

### Citation List

### Patent Literatures

[Patent Literature 1] JP5960729B
[Patent Literature 2] JP6415326B
[Patent Literature 3] JP2014-155455A

### Summary of Invention

### Technical Problem

The objective of the present invention is to provide a transformant of a *Hydrogenophilus* bacterium, in other words, a transformed *Hydrogenophilus* bacterium, that is capable of efficiently producing crotyl alcohol utilizing carbon dioxide as a sole carbon source, and a method for efficiently producing crotyl alcohol using this transformant.

### Solution to Problem

In order to achieve the above objective, the present inventors have conducted a lot of research and have obtained the following findings:
(i) *Hydrogenophilus* bacteria do not originally produce crotyl alcohol, as they do not have genes encoding enzymes that catalyze the crotyl alcohol production reaction. Therefore, it is necessary to introduce genes encoding enzymes that catalyze this reaction to *Hydrogenophilus* bacteria in order to provide crotyl alcohol-producing capability to the *Hydrogenophilus* bacteria.
(ii) When a gene encoding an enzyme having crotonaldehyde dehydrogenase activity, which produces crotonaldehyde from crotonyl-CoA (hereinafter, sometimes, referred to as "crotonaldehyde dehydrogenase") and a gene encoding an enzyme having crotyl alcohol dehydrogenase activity, which produces crotyl alcohol from crotonaldehyde (hereinafter, sometimes, referred to as "crotyl alcohol dehydrogenase") are introduced into *Hydrogenophilus* bacteria, the introduced genes are expressed within the *Hydrogenophilus* bacteria. As a result, the *Hydrogenophilus* bacteria become capable of producing crotyl alcohol.

The same effect can be achieved by introducing a gene encoding a multifunctional enzyme having both crotonaldehyde dehydrogenase activity, which produces crotonaldehyde from crotonyl-CoA and crotyl alcohol dehydrogenase activity, which produces crotyl alcohol from crotonaldehyde (hereinafter, sometimes, referred to as "crotonaldehyde-crotyl alcohol dehydrogenase"), instead of introducing above two-kind genes.

The present invention has been completed based on the above findings and provides the following aspects [1]-[5]:
[1] A transformant (i.e., a transformed bacterium) obtained by introducing into a *Hydrogenophilus* bacterium any one of the following genes (1) to (5):
   (1) A gene encoding an enzyme having crotonaldehyde dehydrogenase activity and a gene encoding an enzyme having crotyl alcohol dehydrogenase activity;
   (2) A gene encoding a multifunctional enzyme having both crotonaldehyde dehydrogenase activity and crotyl alcohol dehydrogenase activity;
   (3) A gene encoding an enzyme having crotonaldehyde dehydrogenase activity and a gene encoding a multifunctional enzyme having both crotonaldehyde dehydrogenase activity and crotyl alcohol dehydrogenase activity;
   (4) A gene encoding an enzyme having crotyl alcohol dehydrogenase activity and a gene encoding a multifunctional enzyme having both crotonaldehyde dehydrogenase activity and crotyl alcohol dehydrogenase activity;
   (5) A gene encoding an enzyme having crotonaldehyde dehydrogenase activity, a gene encoding an enzyme having crotyl alcohol dehydrogenase activity, and a gene encoding a multifunctional enzyme having both crotonaldehyde dehydrogenase activity and crotyl alcohol dehydrogenase activity.
[2] The transformant according to [1], wherein the gene encoding a multifunctional enzyme having both crotonaldehyde dehydrogenase activity and crotyl alcohol dehydrogenase activity is the following DNA (a), (b), (c), (d), or (e):
   (a) A DNA comprising the nucleotide sequence of SEQ ID NO: 1;
   (b) A DNA comprising a nucleotide sequence having 90% or more identity with SEQ ID NO: 1 and encoding a polypeptide having both crotonaldehyde dehydrogenase activity and crotyl alcohol dehydrogenase activity;
   (c) A DNA encoding a polypeptide comprising the amino acid sequence of SEQ ID NO: 2;
   (d) A DNA encoding a polypeptide comprising an amino acid sequence having 90% or more identity with SEQ ID NO: 2 and having both crotonaldehyde dehydrogenase activity and crotyl alcohol dehydrogenase activity;
   (e) A DNA encoding a polypeptide comprising an amino acid sequence in which 1 to 80 amino acids are deleted, substituted, inserted, or added in the amino acid sequence of SEQ ID NO: 2, and having both crotonaldehyde dehydrogenase activity and crotyl alcohol dehydrogenase activity.
[3] The transformant according to [1] or [2], wherein the gene encoding an enzyme having crotyl alcohol dehydrogenase activity is the following DNA (f), (g), (h), (i), or (j):
   (f) A DNA comprising the nucleotide sequence of SEQ ID NO: 3, 5, 7, 9, 11, 13, 15, or 17;
   (g) A DNA comprising a nucleotide sequence having 90% or more identity with SEQ ID NO: 3, 5, 7, 9, 11, 13, 15, or 17, and encoding a polypeptide having crotyl alcohol dehydrogenase activity;
   (h) A DNA encoding a polypeptide comprising the amino acid sequence of SEQ ID NO: 4, 6, 8, 10, 12, 14, 16, or 18;
   (i) A DNA encoding a polypeptide comprising an amino acid sequence having 90% or more identity with SEQ ID NO: 4, 6, 8, 10, 12, 14, 16, or 18, and having crotyl alcohol dehydrogenase activity;
   (j) A DNA encoding a polypeptide comprising an amino acid sequence in which 1 to 30 amino acids are deleted, substituted, inserted, or added in the amino acid sequence of SEQ ID NO: 4, 6, 8, 10, 12, 14, 16, or 18, and having crotyl alcohol dehydrogenase activity.
[4] The transformant according to any one of [1] to [3], wherein the *Hydrogenophilus* bacterium is *Hydrogenophilus thermoluteolus.*
[5] A method for producing crotyl alcohol, comprising culturing the transformant according to any one of [1] to [4] using substantially only carbon dioxide as a carbon source.

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical product raw materials can be produced while suppressing global warming attributed to increased carbon dioxide emissions.

Hydrogen-oxidizing bacteria that can grow by utilizing the chemical energy generated by the reaction of hydrogen with oxygen and by using carbon dioxide as a sole carbon source, can produce chemical products from a mixture of oxygen, hydrogen, and carbon dioxide gases as raw material. Therefore, the bacteria can achieve the efficient conversion of carbon dioxide into organic compounds and can be cultured in a simple culture medium. Typically, hydrogen-oxidizing bacteria grow slowly, but the growth rate of *Hydrogenophilus* bacteria among hydrogen-oxidizing bacteria is exceptionally high. The Journal of Mitsubishi Research Institute No. 34 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

*Hydrogenophilus* bacteria originally possess genes encoding enzymes that catalyze the production of crotonyl-CoA from pyruvic acid. However, they do not have genes encoding enzymes that catalyze the subsequent conversion of crotonyl-CoA to crotyl alcohol.

The present inventor has found that when a heterologous gene is introduced into *Hydrogenophilus* bacteria, it is difficult to express functional proteins. Nevertheless, according to the present invention, by introducing a gene encoding an enzyme having crotonaldehyde dehydrogenase activity that catalyzes the production of crotonaldehyde from crotonyl-CoA and a gene encoding an enzyme with crotyl alcohol dehydrogenase activity that catalyzes the production of crotyl alcohol from crotonaldehyde into *Hydrogenophilus* bacteria, these genes can be expressed in the *Hydrogenophilus* bacteria. As a result, the transformed *Hydrogenophilus* bacteria are capable of producing crotyl alcohol using carbon dioxide as a sole carbon source.

Same results are obtained when a gene encoding a multifunctional enzyme having crotonaldehyde dehydrogenase activity and crotyl alcohol dehydrogenase activity is introduced into *Hydrogenophilus* bacteria, instead of introducing above two-kind genes into *Hydrogenophilus* bacteria.

As described above, *Hydrogenophilus* bacteria have an atypically remarkable carbon dioxide fixation ability among organisms having carbon dioxide fixation ability. Therefore, using the transformant of the present invention enables industrial-scale production of crotyl alcohol through fixation of carbon dioxide.

Crotyl alcohol can be used as an intermediate in the production of butadiene. The crotyl alcohol produced by using the transformant of the present invention can be used as a raw material of butadiene production via microbial processes. The crotyl alcohol can also be used as a raw material of butadiene synthesis through an enzymatic or chemical reaction.

### Brief Description of Drawings

FIG. 1 is a diagram illustrating a pathway in which crotonyl-CoA is converted to butadiene through the introduction of exogenous genes, in addition to the metabolic pathway of production of crotonyl-CoA from pyruvic acid, which is commonly found in many microorganisms.
FIG. 2 is a chromatogram of gas chromatography showing that the crude enzyme produced by the transformant prepared in "Examples" produced crotyl alcohol, using crotonyl-CoA as a substrate.
FIG. 3 is a chromatogram of gas chromatography showing that the crude enzyme produced by the transformant prepared in "Examples" produced crotyl alcohol, using crotonaldehyde as a substrate.

### Mode for Carrying Out the Invention

Hereinafter, the present invention will be described in details.

### (1) Transformant having crotyl alcohol production capability

The transformant of the present invention is that obtained by introducing any one of the genes (1) to (5) described below into a *Hydrogenophilus* bacterium as a host. In other words, the transformant of the present invention has one or more exogenous genes of the genes (1) to (5) described below.
(1) A gene encoding an enzyme having crotonaldehyde dehydrogenase activity and a gene encoding an enzyme having crotyl alcohol dehydrogenase activity;
(2) A gene encoding a multifunctional enzyme having both crotonaldehyde dehydrogenase activity and crotyl alcohol dehydrogenase activity;
(3) A gene encoding an enzyme having crotonaldehyde dehydrogenase activity and a gene encoding a multifunctional enzyme having both crotonaldehyde dehydrogenase activity and crotyl alcohol dehydrogenase activity;
(4) A gene encoding an enzyme having crotyl alcohol dehydrogenase activity and a gene encoding a multifunctional enzyme having both crotonaldehyde dehydrogenase activity and crotyl alcohol dehydrogenase activity;
(5) A gene encoding an enzyme having crotonaldehyde dehydrogenase activity, a gene encoding an enzyme having crotyl alcohol dehydrogenase activity, and a gene encoding a multifunctional enzyme having both crotonaldehyde dehydrogenase activity and crotyl alcohol dehydrogenase activity.

The gene of crotonaldehyde-crotyl alcohol dehydrogenase, the crotonaldehyde dehydrogenase gene, and the crotyl alcohol dehydrogenase gene may be the DNA of a gene isolated from naturally occurring bacteria, or may be a DNA artificially synthesized using methods known to those skilled in the art.

Each of the gene of crotonaldehyde-crotyl alcohol dehydrogenase, crotonaldehyde dehydrogenase gene, and crotyl alcohol dehydrogenase gene may be introduced individually or in combination of two or more. The present invention also encompasses transformants into which genes other than the genes (1) to (5) described above are introduced.

### Gene of crotonaldehyde-crotyl alcohol dehydrogenase

The gene encoding crotonaldehyde-crotyl alcohol dehydrogenase does not necessarily have to be known as a gene identified as a gene of crotonaldehyde-crotyl alcohol dehydrogenase. It is sufficient that the gene encodes a multifunctional enzyme that has both crotonaldehyde dehydrogenase activity, which produces crotonaldehyde from crotonyl-CoA, and crotyl alcohol dehydrogenase activity, which produces crotyl alcohol from crotonaldehyde. This multifunctional enzyme may be a bifunctional enzyme that possesses only crotonaldehyde dehydrogenase activity and crotyl alcohol dehydrogenase activity.

An example of a gene of crotonaldehyde-crotyl alcohol dehydrogenase that can be used in the present invention is the gene derived from *Clostridium akagii,* consisting of the nucleotide sequence of SEQ ID NO: 1. The crotonaldehyde-crotyl alcohol dehydrogenase from *Clostridium akagii* is a bifunctional enzyme possessing both crotonaldehyde dehydrogenase activity and crotyl alcohol dehydrogenase activity. It is confirmed that this bifunctional enzyme from *Clostridium akagii* functions in *Hydrogenophilus* bacteria based on data shown in "Examples" that a crude enzyme produced intracellularly by *Clostridium akagii* produced crotyl alcohol using crotonyl-CoA as a substrate in the presence of NADH.

In the present invention, DNA comprising (in particular, consisting of) the nucleotide sequence of SEQ ID NO:1 may be used.

Furthermore, DNA comprising (particularly consisting of) a nucleotide sequence having 90% or more, especially 95% or more, especially 98% or more, and especially 99% or more identity with SEQ ID NO: 1, and encoding a polypeptide having crotonaldehyde dehydrogenase activity and crotyl alcohol dehydrogenase activity, may also be used.

As the gene encoding crotonaldehyde-crotyl alcohol dehydrogenase, DNA encoding a crotonaldehyde-crotyl alcohol dehydrogenase from *Clostridium akagii,* consisting of the amino acid sequence of SEQ ID NO: 2, may be used.

The present invention may also employ DNA encoding a polypeptide comprising (particularly consisting of) the amino acid sequence of SEQ ID NO:2.

DNA encoding a polypeptide comprising (particularly consisting of) an amino acid sequence having 90% or more, especially 95% or more, especially 98% or more, and especially 99% or more identity with SEQ ID NO: 2 and having crotonaldehyde dehydrogenase activity and crotyl alcohol dehydrogenase activity, may also be used.

Furthermore, DNA encoding a polypeptide comprising (particularly consisting of) an amino acid sequence in which 1 to 100, 1 to 80, 1 to 50, 1 to 30, 1 to 10, 1 to 5, 1 to 4, 1 to 3, 1 to 2, or 1 amino acids are deleted, substituted, inserted, or added in SEQ ID NO: 2, and having crotonaldehyde dehydrogenase activity and crotyl alcohol dehydrogenase activity, may also be used.

In the present invention, the fact that a test polypeptide exhibits crotonaldehyde dehydrogenase activity using crotonyl-CoA as a substrate and crotyl alcohol dehydrogenase activity using crotonaldehyde as a substrate is determined by reacting the test polypeptide with crotonyl-CoA in the presence of NADH and detecting the resulting crotyl alcohol using gas chromatograph or the like.

### Gene of crotonaldehyde dehydrogenase

The gene encoding an enzyme having crotonaldehyde dehydrogenase activity does not necessarily have to be known as a gene identified as crotonaldehyde dehydrogenase gene, as long as the gene encodes a polypeptide having crotonaldehyde dehydrogenase activity.

In the present invention, the fact that a test polypeptide has crotonaldehyde dehydrogenase activity using crotonyl-CoA as a substrate is determined by reacting the test polypeptide with crotonyl-CoA in the presence of NADH and detecting the resulting crotonaldehyde using gas chromatograph or the like.

In the present invention, as the crotonaldehyde dehydrogenase gene, a gene encoding an aldehyde-alcohol dehydrogenase in which the aldehyde dehydrogenase domain is capable of producing crotonaldehyde from crotonyl-CoA, but the alcohol dehydrogenase domain is unable to produce crotyl alcohol from crotonaldehyde can be used. Although this enzyme is generally referred to as an "aldehyde-alcohol dehydrogenase," it is classified as a crotonaldehyde dehydrogenase in the present invention.

In the present invention, the fact that a test polypeptide has aldehyde dehydrogenase activity using crotonyl-CoA as a substrate is determined by reacting the test polypeptide with crotonyl-CoA in the presence of NADH, and detecting the resulting crotonaldehyde using gas chromatograph or the like.

### Gene of crotyl alcohol dehydrogenase

A gene encoding an enzyme having crotyl alcohol dehydrogenase activity does not need to be known as a gene identified as a crotyl alcohol dehydrogenase gene, as long as the gene encodes a polypeptide capable of producing crotyl alcohol from crotonaldehyde.

Examples of crotyl alcohol dehydrogenase genes that can be used in the present invention include those listed in Table 1. Table 1 shows the source microorganisms of enzymes, the nucleotide sequences of genes encoding the enzymes, and the amino acid sequences of the enzymes.

**[Table 1]**

| Origin of crotyl alcohol dehydrogenase gene | Nucleotide sequence | Amino acid sequence |
|---|---|---|
| *Bacillus coagulans* | SEQ ID NO:3 | SEQ ID NO:4 |
| *Zymomonas mobilis subsp.pomaceae* | SEQ ID NO:5 | SEQ ID NO:6 |
| *Thermoclostridium caenicola* | SEQ ID NO:7 | SEQ ID NO:8 |
| *Thermoanaerobacter mathranii* | SEQ ID NO:9 | SEQ ID NO:10 |
| *Thermoanaerobacter ethanolicus* | SEQ ID NO:11 | SEQ ID NO:12 |
| *Bacillus coagulans* | SEQ ID NO:13 | SEQ ID NO:14 |
| *Geobacillus thermoglucosidasius* | SEQ ID NO:15 | SEQ ID NO:16 |
| *Fonticella tunisiensis* | SEQ ID NO:17 | SEQ ID NO:18 |

The crotyl alcohol dehydrogenases consisting of the amino acid sequences of SEQ ID NOs: 4 and 6, derived from *Bacillus coagulans* and *Zymomonas mobilis* subsp. *pomaceae,* respectively, are not multifunctional enzymes possessing aldehyde dehydrogenase activity.

The crotyl alcohol dehydrogenases consisting of the amino acid sequences of SEQ ID NOs: 8, 10, 12, 14, 16, and 18, derived from *Thermoclostridium caenicola, Thermoanaerobacter mathranii, Thermoanaerobacter ethanolicus, Bacillus coagulans, Geobacillus thermoglucosidasius,* and *Fonticella tunisiensis,* respectively, belong to aldehyde-alcohol dehydrogenases. However, the aldehyde dehydrogenase domains of these enzymes are not capable of producing crotonaldehyde from crotonyl-CoA.

In the present invention, DNA comprising (particularly consisting of) a nucleotide sequence having 90% or more, especially 95% or more, especially 98% or more, and especially 99% or more identity with SEQ ID NO: 3, 5, 7, 9, 11, 13, 15, or 17, and encoding a polypeptide having crotyl alcohol dehydrogenase activity, may also be used.

DNA encoding a polypeptide that comprises (particularly consists of) an amino acid sequence having 90% or more, especially 95% or more, especially 98% or more, and especially 99% or more identity with the amino acid sequence of SEQ ID NO: 4, 6, 8, 10, 12, 14, 16, or 18, and having crotyl alcohol dehydrogenase activity, may also be used.

Furthermore, DNA encoding a polypeptide that comprises (particularly consists of) an amino acid sequence in which 1 to 100, 1 to 90, 1 to 80, 1 to 50, 1 to 30, 1 to 10, 1 to 5, 1 to 4, 1 to 3, 1 to 2, or 1 amino acids are deleted, substituted, inserted, or added in the amino acid sequence of SEQ ID NO: 4, 6, 8, 10, 12, 14, 16, or 18, and has crotyl alcohol dehydrogenase activity, may also be used.

As described above, in the present invention, a gene encoding aldehyde-alcohol dehydrogenase of which alcohol dehydrogenase domain is capable of producing crotyl alcohol from crotonaldehyde, but the aldehyde dehydrogenase domain is not capable of producing crotonaldehyde from crotonyl-CoA, may also be used as the gene of an enzyme having crotyl alcohol dehydrogenase activity. Although such an enzyme is generally referred to as "aldehyde-alcohol dehydrogenase", it is classified as crotyl alcohol dehydrogenase in the present invention.

In the present invention, the fact that a test polypeptide has crotyl alcohol dehydrogenase activity using crotonaldehyde as a substrate is determined by reacting the test polypeptide with crotonaldehyde in the presence of NADH, and detecting the resulting crotyl alcohol using gas chromatograph or the like.

DNA sequences encoding a protein that include the amino acid sequence of SEQ ID NO: 2, 4, 6, 8, 10, 12, 14, 16, or 18 may contain various nucleotide substitutions within the coding region due to codon degeneracy, or may contain various nucleotide substitutions considering codon preferences in *Hydrogenophilus* bacteria, as long as the substitutions do not alter the amino acid sequence of the protein expressed from the coding region.

In the present invention, the identities of nucleotide sequences and amino acid sequences are calculated using GENETYX version 17 (GENETYX Corporation).

In the present invention, a DNA comprising a nucleotide sequence having an identity of 90% or more and less than 100% with the nucleotide sequence of a certain DNA and encoding a polypeptide having the same type activity as the polypeptide encoded by the certain DNA is referred to as the "homolog" of the DNA. A polypeptide comprising an amino acid sequence having an identity of 90% or more and less than 100% with the amino acid sequence of a certain polypeptide and having the same type activity as the certain polypeptide is referred to as the "homolog" of the certain polypeptide. A polypeptide comprising an amino acid sequence in which 1 to 100 amino acids are deleted, substituted, inserted, or added in the amino acid sequence of a certain polypeptide and having the same type activity as the certain polypeptide is referred to as the "homolog" of the certain polypeptide.

### Hydrogenophilus bacteria

Examples of *Hydrogenophilus* bacteria include *Hydrogenophilus thermoluteolus, Hydrogenophilus halorhabdus, Hydrogenophilus denitrificans, Hydrogenophilus hirschii, Hydrogenophilus islandicus, Hydrogenophilus thiooxidans, Hydrogenophilus* bacterium strain Mar3 (*Hydrogenophilus* sp. Mar3), and *Hydrogenophilus* bacterium strain Z1038 (*Hydrogenophilus* sp. 21038). In particular, *Hydrogenophilus thermoluteolus* is preferable because its superior growth rate enables top-level carbon dioxide fixation among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977)) (It proliferates double per hour.). *Hydrogenophilus thermoluteolus* strain NBRC 14978 is internationally deposited under the Budapest Treaty, and is thus publicly available.

The *Hydrogenophilus* bacteria as hosts is to be introduced in the present invention may be bacteria isolated from nature or genetically modified bacteria derived from bacteria isolated from nature. Genetic modification may be intended, for example, to allow high expression of an introduced gene.

Such a genetic modification can be made, for example, by removing (curing) endogenous plasmids in *Hydrogenophilus* bacteria. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### Methods for producing transformants

Next, methods for obtaining transformants by introducing genes of above mentioned (1) to (5) into *Hydrogenophilus* bacteria are described.

The introduction of these genes into *Hydrogenophilus* bacteria can be carried out using common methods for introducing exogenous genes into bacteria. These genes may be directly introduced into *Hydrogenophilus* bacteria. Alternatively, they may be incorporated into a vector for transformation such as a plasmid vector, viral vector, cosmid, fosmid, BAC, or YAC, and then, an obtained vector may be introduced into *Hydrogenophilus* bacteria.

Vectors for transformation should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), vectors obtained by genetically modifying these vectors (For example, pCAMO-6), and the like.

Among them, pCAMO-6 is preferable. Those skilled in the art can prepare pCAMO-6 according to "Examples" of this specification.

Examples of promoters in vectors include tac promoter, lac promoter, trc promoter, or each of promoters OXB1 and OXB11 to OXB20 from Oxford Genetics Ltd. Examples of terminators in vectors include the T1T2 terminator of *Escherichia coli* rRNA operon rrnB, the t0 transcription terminator of bacteriophage λ, T7 terminator, and the like.

Introduction of these genes into a *Hydrogenophilus* bacterium (transformation) can be carried out by publicly known methods such as calcium chloride method, calcium phosphate method, DEAE-dextran transfection method, and electric pulse method (electroporation method).

### (2) Method for producing crotyl alcohol

The present invention provides a method for producing crotyl alcohol with use of the transformant of the present invention described above. The method includes a step of culturing the transformant of the present invention using an inorganic or organic culture medium while supplying a gas containing carbon dioxide, preferably, a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that crotyl alcohol can be produced efficiently.

*Hydrogenophilus* bacteria can grow using hydrogen as a source of energy and using carbon dioxide as a sole carbon source, and thus, carbon dioxide can be fixed efficiently particularly by producing crotyl alcohol by using substantially only carbon dioxide (in particular, by using only carbon dioxide) as a carbon source. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using substantially only carbon dioxide as a carbon source" and "Using only carbon dioxide as a carbon source" encompass cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and crotyl alcohol can be produced efficiently.

When batch culture is utilized, mixed gases can be entrapped within an airtight culture container and static culture or shaking culture can be carried out. Shaking culture is preferable in that better dissolution of mixed gases into the culture medium can be achieved. When continuous culture is utilized, mixed gases can be continuously supplied into an airtight culture container and shaking culture can be carried out, or the transformant can be cultured using an airtight culture container while introducing mixed gases into the culture medium by bubbling.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and crotyl alcohol can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and crotyl alcohol can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and crotyl alcohol can be produced efficiently.

Crotyl alcohol is produced in the medium solution by culturing the transformant in the above-described manner. Collecting the medium solution will enable the recovery of crotyl alcohol, however, crotyl alcohol can furthermore be separated from the medium solution by publicly known methods. Such publicly known methods include membrane separation, distillation, and the like.

### Examples

The present invention will now be described with reference to examples. The technical scope of the present invention is not limited by the following examples.

### (1) Construction of plasmid vector (pCAMO-6)

The method for constructing a pCAMO-6 plasmid vector used for the introduction of genes is described below.

### (1-1) Preparation of tac promoter-containing DNA fragment

A pMAL-c5X plasmid manufactured by New England Biolabs was used as a template to amplify a DNA fragment containing a tac promoter by PCR using a pair of primers shown below. Unless otherwise noted, PCR described below was performed in the usual manner using 2720 Thermal Cycler manufactured by Thermo Fisher Scientific Inc. and using KOD FX Neo (manufactured by Toyobo Co., Ltd.) as a reaction reagent.

Primers for amplification of tac promoter
(a-1) 5'-TTTTATAACCCGGGCCATCGACTGCACGGTGCACC-3' (SEQ ID NO: 19)
(b-1) 5'-TGCTAGCACTGTTTCCTGTGTGAAATTGTTATCCG-3' (SEQ ID NO: 20)

The primer (a-1) contains a SmaI restriction enzyme site as indicated by underlining.

The resulting reaction mixture was subjected to electrophoresis on an agarose gel. The results showed that an about 0.3-kbp DNA fragment corresponding to the tac promoter was detected. A gel portion containing the DNA fragment was cut out of the agarose gel, and the gel portion was freeze-thawed to collect the DNA fragment.

### (1-2) Preparation of multicloning site-containing DNA fragment

For the preparation of a DNA fragment containing a multicloning site, the former and latter halves of the multicloning site were separately produced by PCR using pairs of primers shown below. This method does not use any template DNA and allows each pair of primers to anneal together in their 3' terminal regions and elongate to form a double-stranded DNA.

Primers for preparation of former half of multicloning site
(a-2) 5'-GGAAACAGTGCTAGCAGATCTGGAGGAGAAAGGCATAT-3' (SEQ ID NO: 21)
(b-2) 5'-

CAGTGCGGCCGCAAGCTTGTCGACGGAGCTCGAATTCGGATCCGATATCAGCATATGCGTTTC
TCCTCCAGA-3′ (SEQ ID NO: 22)

The 3' terminal 20-nucleotide sequences of the primers (a-2) and (b-2) are complementary to each other.

Primers for preparation of latter half of multicloning site

(a-3) 5′-
ACAAGCTTGCGGCCGCACTGCAGCACCATCACCACCATCATTGATAAGATCCGGCTGCTAACA
AAGCCCGAAAGGAAGCT-3′ (SEQ ID NO: 23)

(b-3) 5′-
TATTTGAATCGAGTTATTGCTCAGCGGTGGCAGCAGCCAACTCAGCTTCCTTTCGGGCTTTGT
-3′ (SEQ ID NO: 24)

The 3' terminal 20-nucleotide sequences of the primers (a-3) and (b-3) are complementary to each other.

The resulting reaction mixture was subjected to electrophoresis on an agarose gel. The results showed that about 0.1-kbp DNA fragments corresponding to the former and latter halves of the multicloning site were detected. The DNA fragments were collected from the gel.

Overlap extension PCR was performed through use of the collected DNA fragments corresponding to the former and latter halves of the multicloning site as a template. The 5' terminal 20-nucleotide sequences of the primers (b-2) and (a-3) used to amplify these template DNA fragments are complementary to each other. For the overlap extension PCR, a combination of the primers (a-2) and (b-3) shown above was used to construct a DNA containing the multicloning site.

The resulting reaction mixture was subjected to electrophoresis on an agarose gel. The results showed that an about 0.2-kbp DNA fragment corresponding to the multicloning site was detected. The DNA fragment was collected from the gel.

### (1-3) Preparation of DNA fragment containing rrnB terminator joined to origin of replication of pUC19

Plasmid pMAL-c5X manufactured by New England Biolabs was used as a template to amplify a DNA fragment containing rrnB terminator by PCR using a pair of primers shown below.

Primers for amplification of rrnB terminator
(a-4) 5'-TAACTCGATTCAAATAAAACGAAAGGCTCAGTCGA-3' (SEQ ID NO: 25)
(b-4) 5'-CCTAGATCCGCGGAGTTTGTAGAAACGCAAAAAGG-3' (SEQ ID NO: 26)

In addition, pUC19 plasmid was used as a template to amplify a DNA fragment containing an origin of DNA replication by PCR using a pair of primers shown below.

Primers for amplification of origin of DNA replication of pUC19
(a-5) 5'-ACAAACTCCGCGGATCTAGGTGAAGATCCTTTTTG-3' (SEQ ID NO: 27)
(b-5) 5'-CGTCCGCGGCCAGCAAAAGGCCAGGAACCGTAAAA-3' (SEQ ID NO: 28)

Each resulting reaction mixture was subjected to electrophoresis on an agarose gel. The results showed that an about 0.3-kbp DNA fragment corresponding to the rrnB terminator and an about 0.8-kbp DNA fragment corresponding to the origin of DNA replication of pUC19 were detected. These DNA fragments were collected from the gel.

Overlap extension PCR was performed through use of the collected DNA fragments corresponding to the rrnB terminator and the origin of DNA replication of pUC19 were used as templates. The 5' terminal 20-nucleotide sequences of the primers (b-4) and (a-5) used to amplify these template DNA fragments are complementary to each other. For the overlap extension PCR, a combination of the primers (a-4) and (b-5) was used to construct a DNA fragment containing the rrnB terminator joined to the origin of replication of pUC19.

The resulting reaction mixture was subjected to electrophoresis on an agarose gel. The results showed that an about 1.0-kbp DNA fragment corresponding to a DNA containing the rrnB terminator joined to the origin of replication of pUC19 was detected. The DNA fragment was collected from the gel

### (1-4) Preparation of DNA fragment containing neomycin/kanamycin resistance gene

Plasmid pK18mobsacB (GenBank: FJ437239.1) (Gene, 145, 69-73 (1994)), which contains a neomycin/kanamycin resistance gene (hereinafter sometimes referred to as "nptII") sequence, was used as a template for PCR. For the amplification of a DNA fragment containing the nptII gene sequence, a pair of primers shown below was used for the PCR.

Primers for amplification of nptII gene
(a-6) 5'-TTGCTGGCCGCGGACGTAGAAAGCCTGTCCGCAGA-3' (SEQ ID NO: 29)
(b-6) 5'-GGCCCGGGTTATAAAAGCCAGTCATTAGGCCTATC-3' (SEQ ID NO: 30)

The primer (b-6) contains a SmaI restriction enzyme site as indicated by underlining.

The resulting reaction mixture was subjected to electrophoresis on an agarose gel. The results showed that an about 1.0-kbp DNA fragment corresponding to the nptII gene was detected. The DNA fragment was collected from the gel.

### (1-5) Construction of circular plasmid

For the DNA fragments prepared in the above (1-1) to (1-4), the terminal region of the DNA fragment in the above (1-1) has a 16-bp sequence homologous to the terminal regions of the DNA fragments in the above (1-4) and (1-2); the terminal region of the DNA fragment in the above (1-2) has a 16-bp sequence homologous to the terminal regions of the DNA fragments in the above (1-1) and (1-3); the terminal region of the DNA fragment in the above (1-3) has a 16-bp sequence homologous to the terminal regions of the DNA fragments in the above (1-2) and (1-4); and the terminal region of the DNA fragment in the above (1-4) has a 16-bp sequence homologous to the terminal regions of the DNA fragments in the above (1-3) and (1-1).

Thus, the DNA fragments in the above (1-1), (1-2), (1-3), and (1-4) can be joined together into the form of a circular DNA by Gibson Assembly (Gibson et al., Nature Methods 6(5):343-345), which is one of DNA ligation methods not using restriction enzymes nor ligases. Gibson Assembly was performed using Gibson Assembly Master Mix (manufactured by New England Biolabs) to join the DNA fragments prepared in the above (1-1) to (1-4) into a circular DNA. With the obtained reaction mixture, *Escherichia coli* JM109 was transformed according to calcium chloride method, and applied to a solid LB medium containing 50 µg/mL kanamycin. The grown strain on the medium was cultured in liquid medium in the usual manner. Plasmid DNA was extracted from the culture liquid and reacted with restriction enzyme, SmaI. This reaction mixture was subjected to electrophoresis on an agarose gel. The results showed that an about 2.3-kbp DNA fragment was observed, which corresponds in size to a single piece of DNA formed by joining the DNA fragments prepared in the above (1-1) to (1-4), demonstrating successful assembly of the DNA fragments.

This circular plasmid DNA, which is replicable in *Escherichia coli,* was named pCAMO-1.

### (1-6) Construction of plasmid vector (pCAMO-4)

*Hydrogenophilus thermoluteolus* TH-1 strain was inoculated with a platinum loop into 5 mL of liquid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, and 4.0 mg of CoCl₂·6H₂O were dissolved in 1 L of distilled water (pH 7.0)]in a test tube. The test tube was filled with a gas mixture of H₂:O₂:CO₂ = 7.5:1:1.5, and the strain was cultured at 50°C.

Endogenous plasmid, pTH1 (Microbiol Resour Announc. 2018 Aug 16; 7(6)), was prepared from the culture liquid according to the conventional alkaline-SDS method.

The prepared pTH-1 of about 66 kbp in size was cut with restriction enzymes ScaI and PvuII, and the pCAMO-1 plasmid was cut with restriction enzyme SmaI. Both of them were ligated together by T4 DNA (manufactured by Takara Bio Inc.).

With the resulting ligation reaction liquid, *Hydrogenophilus thermoluteolus* TH-1 strain (NBRC 14978) was transformed according to electric pulse method (electroporation), applied to solid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, 4.0 mg of CoCl₂·6H₂O, and 15 g of agar were dissolved in 1 L of distilled water (pH 7.0)] containing 50 µg/mL kanamycin, and cultured in a chamber filled with a gas mixture of H₂:O₂:CO₂ = 7.5:1:1.5 at 50°C for 60 hours.

Each of seven strains grown on the solid A medium was inoculated with a platinum loop into 5 mL of liquid A medium containing 50 µg/mL kanamycin in a test tube. The test tubes were filled with a gas mixture of H₂:O₂:CO₂ = 7.5:1:1.5, and the strains were cultured with shaking at 52°C. Plasmid DNA was extracted from the culture and subjected to electrophoresis on an agarose gel. The results showed that all of the 7 strains had the same plasmid DNA of about 5.5 kbp in size.

The extracted plasmid was used as a template to amplify a DNA fragment containing the endogenous plasmid pTH1 inserted in the SmaI restriction enzyme site of pCAMO-1 by PCR using a pair of primers shown below. The pair of primers correspond to the regions at both sides of the SmaI restriction enzyme site of pCAMO-1.

Primers for amplification of inserted pTH1 DNA fragment
(a-7) 5'-AATTGTCAGATAGGCCTAATGACTGGCTTTTATAA-3' (SEQ ID NO: 31)
(b-7) 5'-TGACGCCAGAAGCATTGGTGCACCGTGCAGTCGAT-3' (SEQ ID NO: 32)

The resulting reaction mixture was subjected to electrophoresis on a 1% agarose gel. The results showed that an about 3.2-kbp DNA fragment was detected. That is, the obtained plasmid contains an about 3.2-kbp DNA fragment corresponding to a part of pTH1, which has been inserted into the SmaI restriction enzyme site of the pCAMO-1 plasmid. This about 3.2-kbp DNA fragment contains the origin of replication that functions in *Hydrogenophilus thermoluteolus* cells, so that the obtained plasmid replicates in *Hydrogenophilus thermoluteolus* cells.

The constructed plasmid was named pCAMO-4.

### (1-7) Construction of plasmid vector (pCAMO-5)

Equal moles of a pair of oligonucleotides shown below were mixed, and the mixture was gently cooled from 98°C to 20°C, thus yielding a double-stranded DNA in which the oligonucleotides were annealed. Both ends of this DNA fragment are equivalent to the protruding ends generated by cleavage with restriction enzymes BglII and NdeI. This DNA fragment and a digested DNA fragment prepared by cutting pCAMO-4 with restriction enzymes BglII and NdeI were ligated together by T4 DNA ligase.
(a-8) 5'-GATCTGGAGGAGAAACGCA-3' (SEQ ID NO: 33)
(b-8) 5'-TATGCGTTTCTCCTCCA-3' (SEQ ID NO: 34)

The constructed plasmid was named pCAMO-5.

### (1-8) Construction of plasmid vector (pCAMO-6)

The endogenous plasmid pTH-1 was used as a template to amplify a DNA fragment containing a mazF gene encoding a plasmid stabilization factor by PCR using a pair of primers shown below.

Primers for amplification of mazF gene
(a-9) 5'-GAGGCCATCTAGGCCATGAGTAAGTCTGACGGAAC-3' (SEQ ID NO: 35)
(b-9) 5'-ATCCGGCACCCATATCTGAACCGGACGCAAACCCG-3' (SEQ ID NO: 36)

The endogenous plasmid pTH-1 was used as a template to amplify a DNA fragment containing a mazE gene encoding a plasmid stabilization factor by PCR using a pair of primers shown below.

Primers for amplification of mazE gene
(a-10) 5'-TTCAGATATGGGTGCCGGATACCCGCCGCCCGGGC-3' (SEQ ID NO: 37)
(b-10) 5'-AAGGCCTTCATGGCCTTATTTCGCGATTCCCAAGA-3' (SEQ ID NO: 38)

The 3' terminal region of the mazF-containing DNA fragment has a 20-bp sequence homologous to the 5' terminal region of the mazE-containing DNA fragment. Thus, the mazF-containing DNA fragment and the mazE-containing DNA fragment can be joined by PCR. The DNA fragments prepared above were mixed, and for the amplification of the joined DNA fragments, the primers (a-9) and (b-10) were used.

The resulting reaction mixture was subjected to electrophoresis on an agarose gel. The results showed that an about 0.7-kbp DNA fragment corresponding to a DNA formed by joining the mazF-containing DNA fragment and the mazE-containing DNA fragment was detected. The DNA fragment was collected from the gel.

The prepared DNA fragment of about 0.7 kbp in size was cut with restriction enzyme SfiI, and the pCAMO-5 plasmid was cut with restriction enzyme SfiI. Both fragments were ligated together by T4 DNA ligase.

The constructed plasmid was named pCAMO-6.

### (2) Construction of gene expression plasmids

Gene expression plasmids were constructed by Gibson Assembly.

DNA fragments of genes derived from bacteria shown below were amplified by PCR using the genome DNA of each bacterium as a template.
Gene of crotonaldehyde-crotyl alcohol dehydrogenase:
   DNA fragment of SEQ ID NO:1 derived from *Clostridium akagii*
Crotyl alcohol dehydrogenase gene:
   DNA fragment of SEQ ID NO:3 derived from *Bacillus coagulans*
   DNA fragment of SEQ ID NO:5 derived from *Zymomonas mobilis subsp.pomaceae*
   DNA fragment of SEQ ID NO:7 derived from *Thermoclostridium caenicola*
   DNA fragment of SEQ ID NO:9 derived from *Thermoanaerobacter mathranii*
   DNA fragment of SEQ ID NO:11 derived from *Thermoanaerobacter ethanolicus*
   DNA fragment of SEQ ID NO:13 derived from *Bacillus coagulans*
   DNA fragment of SEQ ID NO:15 derived from *Geobacillus thermoglucosidasius*
   DNA fragment of SEQ ID NO:17 derived from *Fonticella tunisiensis*

The primers shown below were used for PCR.
Primers for amplification of gene of crotonaldehyde-crotyl alcohol dehydrogenase of *Clostridium akagii* (SEQ ID NO:1):
(a-11) 5'-GATCTGGAGGAGAAACGCATATGAAAGTAACAAACATAGAAGAG-3' (SEQ ID NO: 39)
(b-11) 5'-TGTCGACGGAGCTCGAATTCTTATTTTTCTCCCATTTTAATTTCACC-3' (SEQ ID NO: 40)

The primers (a-11) and (b-11) contain their respective sequences homologous to sequences in the pCAMO-6 vector.
Primers for amplification of crotyl alcohol dehydrogenase gene of *Bacillus coagulans* (SEQ ID NO:3):
(a-12) 5'-GATCTGGAGGAGAAACGCATATGAGAGCAGCAGTCGTCAGTTCT-3' (SEQ ID NO: 41)
(b-12) 5'-TGTCGACGGAGCTCGAATTCTTATTTCAACGACATATCCAGGAC-3' (SEQ ID NO: 42)

The primers (a-12) and (b-12) contain their respective sequences homologous to sequences in the pCAMO-6 vector.
Primers for amplification of crotyl alcohol dehydrogenase gene of *Zymomonas mobilis subsp.pomaceae* (SEQ ID NO:5):
(a-13) 5'-GATCTGGAGGAGAAACGCATATGAAAGCAGCCGTCATAACTAAA-3' (SEQ ID NO: 43)
(b-13) 5'-TGTCGACGGAGCTCGAATTCCTAGTGATGGGTAAAATCAACAAC-3' (SEQ ID NO: 44)

The primers (a-13) and (b-13) contain their respective sequences homologous to sequences in the pCAMO-6 vector.
Primers for amplification of crotyl alcohol dehydrogenase gene of *Thermoclostridium caenicola* (SEQ ID NO:7):
(a-14) 5'-GATCTGGAGGAGAAACGCATATGGAAAACAAAGAAAGAAAGATT-3' (SEQ ID NO: 45)
(b-14) 5'-TGTCGACGGAGCTCGAATTCTCATGCTCTCTTCGCTCCGACTCT-3' (SEQ ID NO: 46)

The primers (a-14) and (b-14) contain their respective sequences homologous to sequences in the pCAMO-6 vector.
Primers for amplification of crotyl alcohol dehydrogenase gene of *Thermoanaerobacter mathranii* (SEQ ID NO:9):
(a-15) 5'-GATCTGGAGGAGAAACGCATATGCCTACTTTATTACAAGAAAAA-3' (SEQ ID NO: 47)
(b-15) 5'-TGTCGACGGAGCTCGAATTCTTATTCTCCATAGGCTTTTCTATA-3' (SEQ ID 48)

The primers (a-15) and (b-15) contain their respective sequences homologous to sequences in the pCAMO-6 vector.
Primers for amplification of crotyl alcohol dehydrogenase gene of *Thermoanaerobacter ethanolicus* (SEQ ID NO:11):
(a-16) 5'-GATCTGGAGGAGAAACGCATATGCCTAACTTATTACAAGAAAGA-3'(SEQ ID NO: 49)
(b-16) 5'-TGTCGACGGAGCTCGAATTCTTATTCTCCATAGGCTTTTCTATA-3' (SEQ ID NO: 50)

The primers (a-16) and (b-16) contain their respective sequences homologous to sequences in the pCAMO-6 vector.

Primers for amplification of crotyl alcohol dehydrogenase gene of *Bacillus coagulans* (SEQ ID NO:13):
(a-17) 5'-GATCTGGAGGAGAAACGCATATGGCAATCGATGAAAAAGTTGTT-3' (SEQ ID NO: 51)
(b-17) 5'-TGTCGACGGAGCTCGAATTCTTATTTTGCTTCAACGCCTTCAAA-3' (SEQ ID NO: 52)

The primers (a-17) and (b-17) contain their respective sequences homologous to sequences in the pCAMO-6 vector.
Primers for amplification of crotyl alcohol dehydrogenase gene of *Geobacillus thermoglucosidasius* (SEQ ID NO:15):
(a-18) 5'-GATCTGGAGGAGAAACGCATATGGCTGTGGAGGAAAGAGTCGTC-3' (SEQ ID NO: 53)
(b-18) 5'-TGTCGACGGAGCTCGAATTCTTAAACTCCTTTAAACGCTTGGCG-3'(SEQ ID NO: 54)

The primers (a-18) and (b-18) contain their respective sequences homologous to sequences in the pCAMO-6 vector.
Primers for amplification of crotyl alcohol dehydrogenase gene of *Fonticella tunisiensis* (SEQ ID NO:17):
(a-19) 5'-GATCTGGAGGAGAAACGCATATGAGGGTTGCAGAACCTATAGGA-3' (SEQ ID NO: 55)
(b-19) 5'-TGTCGACGGAGCTCGAATTCTTATTTATTATTTTCTTCAGTTTC-3' (SEQ ID NO: 56)

The primers (a-19) and (b-19) contain their respective sequences homologous to sequences in the pCAMO-6 vector.

The resulting reaction mixtures were subjected to electrophoresis on an agarose gel. The results showed that about 1.0-kbp DNA fragments for crotyl alcohol dehydrogenase genes and about 2.7-kbp DNA fragment for crotonaldehyde-crotyl alcohol dehydrogenase gene were detected.

Each of gel portions containing these DNA fragments was cut out of the agarose gel, and the DNA fragments were collected from the gel portions with GEL/PCR Purification Mini Kit (FAVORGEN).

The pCAMO-6 plasmid vector was amplified by PCR for DNA ligation by Gibson Assembly. The primers shown below were used for PCR.
Primers for amplification of pCAMO-6 plasmid vector
(a-20) 5'-GAATTCGAGCTCCGTCGACA-3' (SEQ ID NO: 57)
(b-20) 5'-ATGCGTTTCTCCTCCAGATC-3' (SEQ ID NO: 58)

The resulting reaction mixture was subjected to electrophoresis on an agarose gel. The results showed that about 5.5-kbp DNA fragment was detected. The gel portion containing the vector DNA fragment was cut out of the agarose gel, and the vector DNA fragment was collected from the gel portion with GEL/PCR Purification Mini Kit (FAVORGEN).

The DNA fragment containing the pCAMO-6 vector as synthesized above and the DNA fragment containing each dehydrogenase gene were joined together with Gibson Assembly Master Mix manufactured by New England Biolabs Inc.

With each of the resulting reaction mixtures, *Escherichia coli* JM109 strain was transformed by heat shock method, applied to LB media containing 50 µg/mL kanamycin, and cultured at 37°C for 24 hours.

Each of the strains grown on the LB media was inoculated with a platinum loop into 5 mL of liquid LB medium containing 50 µg/mL kanamycin in a test tube, and cultured with shaking at 37°C. Plasmid DNA was extracted from the culture liquid. The sequence of the gene inserted in each plasmid was analyzed by the Sanger method by Eurofins Genomics K.K. under contract, and confirmed to be identical to the corresponding sequence in a database.

### (3) Transformants of Hydrogenophilus thermoluteolus

### (3-1) Gene transfer

*Hydrogenophilus thermoluteolus* TH-1 strain was transformed with each of the obtained plasmids by the electric pulse method (electroporation), applied to solid LB media containing 50 µg/mL kanamycin, and cultured at 52°C for 24 hours. The strains grown on the solid LB media were inoculated with a platinum loop on separate solid LB media containing 50 µg/mL kanamycin, and cultured at 52°C for 24 hours. The grown strains on the solid LB media were examined to confirm whether the inserted fragments in the respective plasmids were amplified by PCR.

The results showed that DNA fragments having a size corresponding to the respective genes were amplified. The *Hydrogenophilus thermoluteolus* TH-1 transformants with the respective plasmids containing the genes of the respective bacteria were named as shown in Table 2.

**[Table 2]**

| Origin of gene | Nucleotide sequence of introduced gene | Transformant |
|---|---|---|
| *Clostridium akagii* | SEQ ID NO: 1 | CA-1 |
| *Bacillus coagulans* | SEQ ID NO: 3 | CA-2 |
| *Zymomonas mobilis subsp.pomaceae* | SEQ ID NO: 5 | CA-3 |
| *Thermoclostridium caenicola* | SEQ ID NO: 7 | CA-4 |
| *Thermoanaerobacter mathranii* | SEQ ID NO: 9 | CA-5 |
| *Thermoanaerobacter ethanolicus* | SEQ ID NO: 11 | CA-6 |
| *Bacillus coagulans* | SEQ ID NO: 13 | CA-7 |
| *Geobacillus thermoglucosidasius* | SEQ ID NO: 15 | CA-8 |
| *Fonticella tunisiensis* | SEQ ID NO: 17 | CA-9 |

### (3-2) Measurement of crotonaldehyde dehydrogenase activity and crotyl alcohol dehydrogenase activity of enzyme produced by transformants

The *Hydrogenophilus thermoluteolus* transformant strain CA-1 as prepared above was inoculated with a platinum loop into liquid A medium containing 50 µg/mL kanamycin, and cultured with shaking for 24 hours while a gas mixture of H₂:O₂:CO₂ = 7.5:1:1.5 was supplied. The cultured bacterial cells were collected by centrifugation (4°C, 5,000 g, 10 minutes) from 2mL of the culture liquid. The bacterial cells were suspended in a 200µL reaction buffer (100mM Tris-HCl(pH7.5), 5µM FeSO₄, 0.2mM DTT) and disrupted by sonication and then centrifuged (4°C, 20,000 g, 10 minutes) to obtain the supernatant of the disrupted cells.

Crotonaldehyde dehydrogenase activity was measured using the disrupted cell supernatant as a crude enzyme solution. That is, the crude enzyme solution was mixed with the reaction buffer, 10mM NADH as a coenzyme, and 1mM crotonyl-CoA as a substrate to proceed a reaction at 52°C. The reaction liquid was analyzed by gas chromatograph (Shimadzu Corporation, GC-2014, Polar-WAX column), and produced crotonaldehyde and crotyl alcohol were identified by detecting peaks having a retention time same as those of respective standard products. Crotyl alcohol is detected when the tested enzyme is a bifunctional enzyme having crotonaldehyde dehydrogenase activity and crotyl alcohol dehydrogenase activity.

Figure 2 shows the chromatogram for the reaction product obtained from crotonyl-CoA and the crude enzyme solution produced by the transformant strain CA-1. Here, NC refers to the negative control strain transformed with an empty vector, and Std indicates the standard products (1 mM each) of crotyl alcohol and crotonaldehyde, respectively.

The crude enzyme produced by transformant strain CA-1, which is obtained by introducing the DNA of SEQ ID NO:1 into *Hydrogenophilus thermoluteolus* TH-1, generated crotyl alcohol from crotonyl-CoA as a substrate. This result indicates that the strain produced a bifunctional enzyme possessing both crotonaldehyde dehydrogenase activity and crotyl alcohol dehydrogenase activity.

Thus, the *Hydrogenophilus thermoluteolus* CA-1 strain produced a functional crotonaldehyde-crotyl alcohol dehydrogenase through growth using carbon dioxide as a sole carbon source. This suggests that the strain is capable of producing crotyl alcohol using carbon dioxide as a sole carbon source.

### (3-3) Measurement of crotyl alcohol dehydrogenase activity of enzyme produced by transformants

The disrupted cell supernatants of *Hydrogenophilus thermoluteolus* transformants CA-2, CA-3, CA-4, CA-5, CA-6, CA-7 and CA-8, which were prepared as above, were used as crude enzyme solutions. The crotyl alcohol dehydrogenase activity of each crude enzyme solutions was assayed. A reaction buffer was added to the crude enzyme solution, then, 10 mM NADH as a coenzyme and 10 mM crotonaldehyde as a substrate were added thereto. The reaction was carried out at 52°C for 15 minutes. The reaction liquid was analyzed by gas chromatograph (Shimadzu Corporation, GC-2014, Polar-WAX column), and produced crotyl alcohol was identified by detecting peaks having a retention time same as that of a standard product.

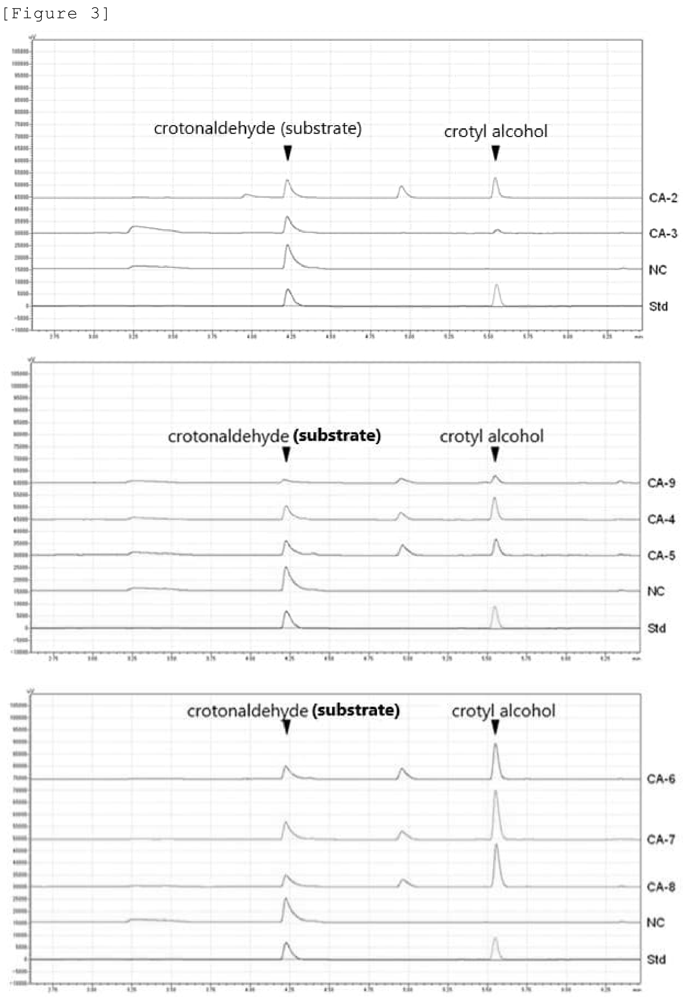

Figure 3 shows the chromatograms for the reaction products obtained from crotonaldehyde and the crude enzyme solutions produced by the respective transformants. Here, NC refers to the negative control strain transformed with an empty vector, and Std indicates the standard products (1 mM each) of crotyl alcohol and crotonaldehyde, respectively.

The crude enzyme solution produced by the transformant which was obtained by introducing the DNA of SEQ ID NOs: 3, 5, 7, 9, 11, 13, 15, or 17 into *Hydrogenophilus thermoluteolus* TH-1 generated crotyl alcohol using crotonaldehyde as a substrate. This indicates that these transformants produced an enzyme having crotyl alcohol dehydrogenase activity.

Thus, the *Hydrogenophilus thermoluteolus* strains CA-2 , CA-3, CA-4, CA-5, CA-6, CA-7, and CA-8 produced functional crotyl alcohol dehydrogenases through growth using carbon dioxide as a sole carbon source. These results suggest that these transformants are capable of producing crotyl alcohol using carbon dioxide as a sole carbon source by further introducing a gene encoding an enzyme possessing crotonaldehyde dehydrogenase activity or a gene encoding a bifunctional enzyme possessing both crotonaldehyde dehydrogenase activity and crotyl alcohol dehydrogenase activity.

### (3-4) Production of crotyl alcohol by transformant

The strain CA-1 prepared as above and the NC strain having the empty vector are inoculated with a platinum loop into separate liquid A media containing 50 µg/mL kanamycin, and cultured with shaking at 52°C for 24 hours while a gas mixture of H₂:O₂:CO₂ = 7.5:1:1.5 is supplied. After that, culture supernatants are collected by centrifugation (4°C, 5,000 g, 10 minutes). Crotyl alcohol is produced in the culture supernatant of the CA-1 strain unlike the culture supernatant of NC strain having the empty vector.

The *Hydrogenophilus* bacterium transformant of the present invention can be prepared by referring to the description of "Examples" of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

SEQ ID Nos: 1 to 18 are shown below.
[Ka 1]
   SEQ ID NO: 1(*Clostridium akagii*) 
ATGAAAGTAACAAACATAGAAGAGTTAAATATAAAGCTCAAAAGTGTAAAAGAAGCTCAAAAAGAGTTTT
CAACCTTTACCCAGGAGATGGTTGATGAAATCTTTAAAAGAGTAGCAATTGCAGCATTAGAAGCCAGAAT
ACCATTGGCTAAAATGGCTGTGGAAGAAAGTGGTATGGGATTACTAGAGGATAAGGTAATAAAAAACCAT
TTTGCTGCTGAATATATATATAATAAATATAAGGATGAAAAAACATGTGGTGTTATTGAAAGAGATGAAA
CCTATGGTATAACAAAAATAGCAGAGCCAATAGGTGTTGTAGCAGCTATAGTACCAACTACCAATCCTAC
CTCTACAGTGATTTTTAAAACACTAATCTCCTTAAAAACTAGAAATGGCATAATATTTTCTCCTCATCCA
AGAGCAAAAAAATCAACAGTAGCAGCTGCAAAAATAATGTTAGAGGCTGCAGTTAAGGCTGGGGCACCTG
AGGGAATAATAGGGTGGATAGACGAACCTTCTATAGAACTTTCAAAAATATTAATGAGTGAAGCAGATGT
AATACTAGCTACAGGAGGACCTAGTATGGTTAAATCGGCTTATTCATCTGGTAAACCAGCTATTGGAGTT
GGACCTGGGAATACACCGTCTATAATAGATGAAAGTGCAGATATACAAATGGCCGTAAGTTCAATAATAT
TATCTAAAACTTATGATAATGGTGTAATATGTGCTTCGGAACAATCTGTAATAGTCTTAGATTCCATATA
TAATAAGGTTAAAAATGAATTCATTAAACGAGGAGCTTATATATTAAGTAAAAATGAGATAGAAAAAGTT
AAAATGATAATGTTTAATAATGGGAATATAAATGCTAGTGTAGTTGGACAATCAGCTTGCAAAATAGCTA
AGTTGGCAGATATTAATGTACCTGAATGGACAAAGGTACTTATTGGAGAAGTTAAGTCTGTGGAATTAGA
AGAACTATTTTCACATGAAAAATTATCCACAGTATTAGCTATGTATAGAGTAAATAGTTTTGATGAAGCT
TTAGAAAAGGCCGAAAGACTGGTGGAATTAGGTGGAAAAGGACATACATCATCATTGTTTATAGATTCTG
TATGTGAAAAAGAAAAAATAGAAAAGTTTAGTTTTAAAATGAAAACTGTAAGAACCTTTATAAATATACC
TTCATCCCAAGGGGCTAGTGGTGATTTATTCAATTTTAAAATAGCACCTTCCTTTACTTTAGGTTGTGGA
TTCTGGGGTGGAAATTCAGTGTCTGAAAACGTAGGACCTAAACATTTGTTGAATATAAAAACAGTAGCTG
AGAGAAGGGAAAATATGCTTTGGTTTAAGGTACCAGAAAAAATATACTTCAAATATGGATGTTTGGAATT
TGCACTTAGGGAATTAAAAGATATGGGTAAGAAAAAAGCATTTATTGTAACTGATAAAGTGCTTTATGAA
TTAGGATATGCCGATAAAGTAACGAAAGTTTTAGAAAAAATGGACATACGGTTTAAAGTTTTTTCAGATG
TTGAGCCAGACCCTACACTTGCCTGTGCGAGTAAAGGCGCCAAAGAAATGCTTGAATTTGAACCAGACAC
TATAATATCCATAGGTGGAGGATCAGCTATGGATGCGGCTAAAATTATGCAAGTTTTATATGAGCATCCA
GAAGAGAAGTTTGATGAACTGGCACTGAAATTTATGGATATAAGAAAGCGAATACACAGTTTTCCAAAGC
TTGGAATTAAGGCTATATCAGTAGCAATACCAACAACTGCTGGTACTGGTTCAGAAGTAACACCTTTTGC
AGTAATAACAGATGAAAAAACGGGGATGAAATATCCACTTTCAGATTATGAGTTAACACCGAATGTGGCA
ATTGTAGATGCAGAATTTATGATGAATATGCCTAAGGGTCTAACTGCGGCAACAGGGATAGATGCACTAA
CTCATGCTATTGAAGCCTATGTATCTGTTATGGCAACGGAATACACAGATGGATTGGCACTTCAAGCAAT
AAAAATGATATTTGAATATTTACCAGCAGCATATAATGAAGGAAAGACTAATATAGTTGCTAGAGAGAAG
ATGGCAAATGCATCTACAATAGCAGGTATGGCGTTCGGAAATGCATTTTTAGGAGTATGTCATTCAATGG
CCCATAAATTAGGAGCCCAGCACCATGTACCCCATGGTATTGCTAATGCAGTGTTAATTGAGGAAGTAAT
TAGATTTAATGCAGTAGACAATCCAGTAAGACAAACTTCATTTCCACAATATAAATATCCAGATGCAAAA
GAAAGATATGCTAGGATTGCTGATTATTTAAAACTAAGTGGAAATACAGATAGTGACAAAGTAGAACAAC
TAATAAAGGCAATTACTAATTTAAAAATAAAATTAAGTATTCCATTGACTATAAAGGATGCAGGAATTAA
AGAAGAAGCTTTTTATTTAACATTGGATGATATGTCTGAGAAGGCTTTTGATGATCAATGCACTAGCGCT
AATCCTAGATATCCATTGATAAGCGAAATTAAACAAATGTTTATAAACGCATTTGGTGAAATTAAAATGG
GAGAAAAATAA
[Ka 2]
   SEQ ID NO: 2(*Clostridium akagii*) 
MKVTNIEELNIKLKSVKEAQKEFSTFTQEMVDEIFKRVAIAALEARIPLAKMAVEESGMG
LLEDKVIKNHFAAEYIYNKYKDEKTCGVIERDETYGITKIAEPIGVVAAIVPTTNPTSTV
IFKTLISLKTRNGIIFSPHPRAKKSTVAAAKIMLEAAVKAGAPEGIIGWIDEPSIELSKI
LMSEADVILATGGPSMVKSAYSSGKPAIGVGPGNTPSIIDESADIQMAVSSIILSKTYDN
GVICASEQSVIVLDSIYNKVKNEFIKRGAYILSKNEIEKVKMIMFNNGNINASVVGQSAC
KIAKLADINVPEWTKVLIGEVKSVELEELFSHEKLSTVLAMYRVNSFDEALEKAERLVEL
GGKGHTSSLFIDSVCEKEKIEKFSFKMKTVRTFINIPSSQGASGDLFNFKIAPSFTLGCG
FWGGNSVSENVGPKHLLNIKTVAERRENMLWFKVPEKIYFKYGCLEFALRELKDMGKKKA
FIVTDKVLYELGYADKVTKVLEKMDIRFKVFSDVEPDPTLACASKGAKEMLEFEPDTIIS
IGGGSAMDAAKIMQVLYEHPEEKFDELALKFMDIRKRIHSFPKLGIKAISVAIPTTAGTG
SEVTPFAVITDEKTGMKYPLSDYELTPNVAIVDAEFMMNMPKGLTAATGIDALTHAIEAY
VSVMATEYTDGLALQAIKMIFEYLPAAYNEGKTNIVAREKMANASTIAGMAFGNAFLGVC
HSMAHKLGAQHHVPHGIANAVLIEEVIRFNAVDNPVRQTSFPQYKYPDAKERYARIADYL
KLSGNTDSDKVEQLIKAITNLKIKLSIPLTIKDAGIKEEAFYLTLDDMSEKAFDDQCTSA
NPRYPLISEIKQMFINAFGEIKMGEK
[Ka 3]
   SEQ ID NO: 3(*Bacillus coagulans*) 
ATGAGAGCAGCAGTCGTCAGTTCTGATAAGCAAAAACTGGTAGAGGTCAAAGATGTGCAG
TTGCGTCCACTTGAATATGGGGAAGCTTTGGTGGATATCGAATACTGCGGTGTCTGCCAT
ACGGATCTGCATGTGGCAAAGGGCGACTTTGGCGATGTGCCGGGACGCATTCTCGGCCAT
GAAGGAGTCGGCATTGTCAGGGAAGTCGCGGATGGTGTCACTTCTCTTAAAGTGGGAGAT
CGTGTAAGCGTTGCCTGGTTTTTTGAAGGCTGCGGGCATTGTGAGTACTGTTTAACGGGA
AATGAAACATTTTGCCGTGATGTGAAAAATGCAGGCTACAGTGTCGACGGAGCAATGGCA
GAGCAGTGCATCGTCAAAGCGGATTACGCCGTAAAAGTGCCGGAAGGGCTGGATCCAAGG
CAGGCGACGAGCATTACTTGCGCAGGTGTCACCACTTATAAAGCCATTAAAGTCGCAGAT
GTGAAGCCGGGCGAATGGATTGCCATTTATGGAGCCGGCGGTCTTGGTAACCTTGCTGTC
CAGTACGCCAAACATGTATTTAATGCGAAAGTCATTGCGATCGATATCAATGACGACAAA
CTTTCACTGGCAAAAGAAGTCGGTGCTGATCTCACCTTTAATTCCAAAATGAAAGGCGAT
CCGGCTGAATGGATCCAAAAAGAAGTTGGCGGCGCGCATGCTGCAGTGGTAACGGCTGTT
TCAAAACAGGCTTTTAACCAGGCTGTCCACTCTGTACGGCCGGTTTCAAAAGTCGTTGCT
GTCGGGCTCCCTCCGGAAACAATGGATCTTGAAATTGTGAAGACGGTTCTTGACGGAATA
GAAGTCGTCGGCTCTTTGGTAGGCACGAGAAAAGACCTTGAGGAAGCCTTTATGTTCGGG
GCGGAAGGAAAAGTCGTCCCGGTTGTGCACACGCGCAGACTGGAAGAAGTGGGAGATGTG
TTTAAAGAAATGGAAGAAGGCAGAATACAGGGCAGGATGGTCCTGGATATGTCGTTGAAA
TAA
[Ka 4]
   SEQ ID NO: 4(*Bacillus coagulans*) 
MRAAVVSSDKQKLVEVKDVQLRPLEYGEALVDIEYCGVCHTDLHVAKGDFGDVPGRILGH
EGVGIVREVADGVTSLKVGDRVSVAWFFEGCGHCEYCLTGNETFCRDVKNAGYSVDGAMA
EQCIVKADYAVKVPEGLDPRQATSITCAGVTTYKAIKVADVKPGEWIAIYGAGGLGNLAV
QYAKHVFNAKVIAIDINDDKLSLAKEVGADLTFNSKMKGDPAEWIQKEVGGAHAAVVTAV
SKQAFNQAVHSVRPVSKVVAVGLPPETMDLEIVKTVLDGIEVVGSLVGTRKDLEEAFMFG
AEGKVVPVVHTRRLEEVGDVFKEMEEGRIQGRMVLDMSLK
[Ka 5]
   SEQ ID NO: 5(*Zymomonas mobilis subsp.pomaceae*) 
ATGAAAGCAGCCGTCATAACTAAAGATCATACGATCGAAGTGAAAGACACCAAATTACGC
CCTCTGAAATACGGGGAAGCGCTTTTGGAAATGGAATATTGCGGGGTATGTCATACCGAT
CTCCACGTGAAAAACGGGGATTTTGGCGATGAAACCGGCAGAATTACCGGCCATGAAGGC
ATCGGTATCGTCAAGCAGGTCGGGGAAGGGGTTACTTCTCTGAAAGTCGGTGACCGCGCC
AGTGTTGCATGGTTCTTCAAAGGCTGCGGCCATTGCGAGTATTGTGTCAGCGGGAATGAA
ACGCTTTGCCGCAACGTTGAAAATGCCGGTTATACGGTTGACGGCGCTATGGCAGAAGAA
TGCATCGTCGTTGCCGATTACTCGGTCAAAGTGCCAGATGGTCTTGATCCTGCGGTTGCC
AGCAGCATCACTTGCGCGGGTGTAACCACCTATAAAGCAGTCAAAGTTTCTCAGATACAG
CCGGGACAATGGCTGGCTATCTATGGCTTGGGCGGTTTAGGCAATCTAGCCCTTCAATAT
GCCAAGAATGTTTTCAACGCCAAAGTGATCGCGATCGATGTCAATGATGAACAGCTCGCT
TTTGCCAAAGAGCTGGGCGCAGATATGGTCATCAATCCGAAAAACGAAGATGCTGCCAAA
ATCATTCAGGAAAAAGTCGGCGGCGCACATGCGACGGTGGTGACGGCTGTTGCCAAATCC
GCCTTTAACTCGGCTGTTGAGGCTATCCGCGCGGGTGGCCGTGTTGTCGCCGTTGGTCTG
CCTCCTGAAAAAATGGATTTGAGCATTCCTCGTTTGGTGCTTGACGGTATCGAAGTCCTA
GGTTCCTTGGTCGGAACGCGGGAAGATTTGAAAGAAGCCTTCCAGTTTGCAGCCGAAGGT
AAGGTCAAACCGAAAGTCACCAAGCGTAAAGTCGAAGAAATCAACCAAATCTTTGACGAA
ATGGAACATGGTAAATTCACAGGCCGTATGGTTGTTGATTTTACCCATCACTAG
[Ka 6]
   SEQ ID NO: 6(*Zymomonas mobilis subsp.pomaceae*) 
MKAAVITKDHTIEVKDTKLRPLKYGEALLEMEYCGVCHTDLHVKNGDFGDETGRITGHEG
IGIVKQVGEGVTSLKVGDRASVAWFFKGCGHCEYCVSGNETLCRNVENAGYTVDGAMAEE
CIVVADYSVKVPDGLDPAVASSITCAGVTTYKAVKVSQIQPGQWLAIYGLGGLGNLALQY
AKNVFNAKVIAIDVNDEQLAFAKELGADMVINPKNEDAAKIIQEKVGGAHATVVTAVAKS
AFNSAVEAIRAGGRVVAVGLPPEKMDLSIPRLVLDGIEVLGSLVGTREDLKEAFQFAAEG
KVKPKVTKRKVEEINQIFDEMEHGKFTGRMVVDFTHH
[Ka 7]
   SEQ ID NO: 7(*Thermoclostridium caenicola*) 
ATGGAAAACAAAGAAAGAAAGATTGTAGACAGTGTGGACGCGTTAAAGAAAGTCATTGCC
CGGGTAAGGGAAGCGCAAAGGAAATTTTCAACCTATTCCCAGGAACAGGTGGATGAAATT
TTTAAGGCTGCTGCCACTGCCGCAAACCAGGCAAGAATTCCGCTGGCGAAAATGGCGGTG
GAAGAAACCGGTATGGGCGTTGTGGAAGATAAAGTTATAAAAAACCACTATGCCGCCGAA
TATGTTTACAATACGTATAAGAACGTAAGAACCTGCGGGGTTTTGGAAGAAGATAAGGCA
TACGGTATCAAAAAAATTGCCGAGCCTGTCGGTGTGATTGCCGCTGTTATTCCTACCACT
AACCCTACTTCCACGGCAATTTTTAAAACCCTGATCGCGCTGAAAACACGCAATGCCGTT
ATTATCAGCCCACATCCGAGAGCTAAAAAATGCACCATCGCTGCGGCAAAAATCGTATTG
GATGCTGCCGTTGCCGCGGGGGCTCCGGAAGGGATTATTGACTGGATTGATGTGCCGAAC
ATTGAGTTGACCAATCTGCTTATGAAAGAAGCAGACATTATCCTTGCCACAGGTGGTCCC
GGTATGGTGAAGGCGGCTTATTCCTCCGGGAAACCGGCTATCGGTGTCGGGCCTGGCAAT
ACTCCGGCCGTTATTGATGAAAGCGCCGACGTTGTCCTTGCCGTGAACTCCATTATTCAT
TCCAAAACGTTTGATAATGGTATGATTTGCGCATCAGAACAATCGGTAATTGTTCACAAG
AATGTTTATGACGCTGTAAAAGCTGAATTTGCGGCCCGTGGATGCTATTTCCTTAAAGAA
GATGAAATTGAAAAGGTACGCAAGACGATTTTTGTCAACGGCGCGTTAAATGCCCAGATT
GTTGGTCAGCCGGCATACAAAATTGCCGAACTTGCCGGTGTGAAAGTGCCTGTAGGCACA
AAAATCCTCATCGGAGAAGTTGACAGCACAGACCCGTCGGAAGAATTTGCCCATGAAAAG
CTGTCACCTGTCCTTGCGATGTATAAAGCTGAGGATATTCATGACGCTTTTGAGAAAGCC
GAACGTTTGGTTGCCAACGGAGGTTACGGTCATACCGCAGCAATTTATTTGGATGTAACC
ACCGAACAGGCTAAAATTGACGAATTCGCTTCGCGTTTGAAGACCTGCCGTATACTTGTG
AATACTCCGTCTTCTCAGGGAGCTATCGGCGACATTTACAACTTTAAGCTTGCGCCGTCG
TTAACTCTTGGCTGTGGTTCCTGGGGCGGAAATTCGGTTTCCGAAAATGTTGGGGTAAAA
CACCTGCTTAATATCAAAACGGTTGTTGAAAGAAGGGAAAATATGCTGTGGTTCCGCGCT
CCCGAAAAAATATACATCAAAAAGGGATGTTTGCCGGTAGCCCTTAATGAGTTGAAATAC
GTCATGGGTAAGAAACGCGCTTTTATAGTTACCGACAGCTTCCTCTATCACAATGGTTAC
ACAAAGCCCATTACCGACAGACTTGACGAACTGGGCATTGAACATACAACGTTTTTTGAT
GTTTTACCCGATCCGACGCTTTCCTGCGCAAAAGCGGGAGCTGAGCAGATGAGAGCCTTC
CAGCCCGATACCATTATTGCCTTCGGTGGCGGATCTGCGATGGACGCCGCGAAAATCATG
TGGGTAATGTATGAGCATCCCGAAGTTGATTTCATGGACATGGCTATGCGTTTTATGGAT
ATCAGGAAAAGGGTTTATACATTTCCGAAAATGGGAGAAAAGGCGTATTTTGTTGCAATA
CCGACTTCTGCGGGTACCGGATCCGAAGTAACTCCTTTTGCGGTTATCACCGACGAAAAG
AGTGGAGTAAAATATCCCCTCGCAGACTACGAGTTGCTCCCCGACATGGCAATTATAGAT
GTGGATTTTCACATGAGTGCTCCCAAGGGCCTTACCGCCGCAGCAGGTATAGATGCCCTC
ACCCATGCGCTTGAGGCGTACGTTTCTCTTATGGCCACTGATTTTACCGACGGACTGGCG
AAACAGGCTCTGCAGATGATTTTTAAATACCTGCCCCGCGCCTATGATAACGGTCAGACC
GATGTGGAAGCACGTGAAAAAATGGCTAATGCCGCCACCATTGCAGGTATGGCGTTTGCA
AATGCTTTCCTTGGTGTTTGCCATTCCATGGCACATAAGCTGGGCGCTTTTTATCATTTG
CCTCATGGTATTGCCAACGCGCTTATGATAGAAGAAGTAATCCGTTTCAACGCTTCGGAA
GCACCGGCCAAAATGGGAACTTTCCCGCAATACGGATACCCGCACGCTTTAAGACGTTAT
GCCGAAATTGCTGAGGCGCTTAATCTCGGCGGCGAAAACGATCATGAAAAGCTGGAAAAT
CTCATTAAAGCCATTAATGATCTGAAGGCAAGGGTGGGGATCAAGTCATCAATCAAAGAG
TATGGCATAAATGAGAAGGAATTCCTGGAACGCCTCGACGAGATGGTTGAACAGGCATTC
GACGACCAGTGCACCGGTACCAATCCGCGATATCCGCTGATGAGCGAAATAAGGCAAATG
TATCTGAATGCTTATTACGGCAAAAAACATTTTGTCGATGAAGAAAAGCCAAATGAAGCG
GATATCGCCGGGACAGAGGATGAAAACAAAGTTAAAAAGGCGTATCTGAGAGCCAGAGTC
GGAGCGAAGAGAGCATGA
[Ka 8]
   SEQ ID NO: 8(*Thermoclostridium caenicola*) 
MENKERKIVDSVDALKKVIARVREAQRKFSTYSQEQVDEIFKAAATAANQARIPLAKMAV
EETGMGVVEDKVIKNHYAAEYVYNTYKNVRTCGVLEEDKAYGIKKIAEPVGVIAAVIPTT
NPTSTAIFKTLIALKTRNAVIISPHPRAKKCTIAAAKIVLDAAVAAGAPEGIIDWIDVPN
IELTNLLMKEADIILATGGPGMVKAAYSSGKPAIGVGPGNTPAVIDESADVVLAVNSIIH
SKTFDNGMICASEQSVIVHKNVYDAVKAEFAARGCYFLKEDEIEKVRKTIFVNGALNAQI
VGQPAYKIAELAGVKVPVGTKILIGEVDSTDPSEEFAHEKLSPVLAMYKAEDIHDAFEKA
ERLVANGGYGHTAAIYLDVTTEQAKIDEFASRLKTCRILVNTPSSQGAIGDIYNFKLAPS
LTLGCGSWGGNSVSENVGVKHLLNIKTVVERRENMLWFRAPEKIYIKKGCLPVALNELKY
VMGKKRAFIVTDSFLYHNGYTKPITDRLDELGIEHTTFFDVLPDPTLSCAKAGAEQMRAF
QPDTIIAFGGGSAMDAAKIMWVMYEHPEVDFMDMAMRFMDIRKRVYTFPKMGEKAYFVAI
PTSAGTGSEVTPFAVITDEKSGVKYPLADYELLPDMAIIDVDFHMSAPKGLTAAAGIDAL
THALEAYVSLMATDFTDGLAKQALQMIFKYLPRAYDNGQTDVEAREKMANAATIAGMAFA
NAFLGVCHSMAHKLGAFYHLPHGIANALMIEEVIRFNASEAPAKMGTFPQYGYPHALRRY
AEIAEALNLGGENDHEKLENLIKAINDLKARVGIKSSIKEYGINEKEFLERLDEMVEQAF
DDQCTGTNPRYPLMSEIRQMYLNAYYGKKHFVDEEKPNEADIAGTEDENKVKKAYLRARV
GAKRA
[Ka 9]
   SEQ ID NO: 9(*Thermoanaerobacter mathranii*) 
ATGCCTACTTTATTACAAGAAAAAAAGGAAACAAAAGAAAAAACAGAAGTAAAAGAAACAACAGATGTTA
AAAAACAAATAGACCTATTAGTAGAAAGGGCAAAAAGAGCCCAAGAAAAATTCATGTCATACACTCAAGA
GCAAATTGATGAAATAGTCAAAGTTATGGCACTAGCGGGCATAGAAAAGCATGTAGAACTGGCAAAATTA
GCTCATGAAGAAACGAAAATGGGGGTTTATGAAGACAAAATAACGAAAAATCTCTTTGCAGTAGAATATG
TGTACAATTATATAAAAGACAAAAAGACTGTCGGCATCCTTAGTGAAAACTTAGAAGAAAATCATATGGA
AGTGGCAGAGCCTGTAGGAATTATAGCAGGGGTTACCCCCGTGACAAACCCTACCTCTACAACAATGTTT
AAATGCTTAATAGCTATCAAAACAAGAAATCCAATAATTTTTAGTTTCCATCCCAAAGCATTAAAATGTA
GTATAGAAGCAGCAAAGACCATGTACGAGGCAGCATTAAAGGCGGGTGCTCCCGAAGGATGCATAGGTTG
GATTGAAACACCTTCAATTGAAGCAACGCAACTTCTCATGACACACCCCGGCGTCTCCCTTATCCTCGCA
ACAGGCGGTGCGGGAATGGTAAAAGCTGCTTACAGCTCAGGAAAACCCGCCCTTGGCGTAGGTCCTGGCA
ATGTCCCCTGCTACATTGAAAAAACTGCAAATATTAAAAGAGCGGTCTCAGACCTAATACTTAGCAAAAC
TTTTGACAATGGCACAGTATGCGCATCAGAACAAGCTGTGATAATAGATGAAGAAATAGCCAATGAAGTA
AAAAAATTAATGAGAGAATATGGTTGCTATTTCTTAAATAAAGAAGAAACTAAAAAGTTAGAAGAATTCG
CTATAGACAAAAACACCGGTTTAATGAATCCAGCAGTTGTGGGTCAGCCTGCAGTGAAAATTGCTCAAAT
GGCTGGATTTAAAGTTCCTGAAAATACCAAAATACTTGTAGCTGAATATCCAGCTGTAGGTCCTAAATAT
CCTCTATCCAGAGAAAAACTAAGCCCAATACTTGCTTTATACACAGTCAAAGATTACAAAGAAGGTATAA
AAAGATGCGAAGAAATGATACAATTCGGCGGACTTGGTCACTCTGCGGTAATACATTCTGAAAACCAACA
AATTATTAATGAATTTGCAAATAGAGTACAAGCAAGTAGAATCCTTGTAAACGCCCCTGCTTCACAAGGC
GCTATTGGAGACATTTATAACACGGCTACTCCATCTTTGACTTTGGGTTGCGGTACAATGGGTGGAAATT
CAACAACAGATAATGTAAGTGTTTATAACCTTATAAACATAAAACGAGTTTTTATAAGAAAAGAGAGAAT
GAAATGGTTCAGAGTTCCACCACAAATCTATTTTGAAAGAGGGTCAATACAGTATCTATCACAAGTAAAA
GGCAAAAAAGCTTTTATTGTAACAGACCCTGCAATGGTTAAATTAGGTTTTGTAGATAAAGTGACATATC
AATTAGATAAAGCAAATATTAAATATGAAATTTTCTCTGAAGTAGAACCAGACCCATCAGTTGACACTGT
AGAAAAAGGGATAAAAATAATGAAGGAGTTCGAGCCTGACTTACTAATAGCTGTAGGTGGTGGTTCTGCA
ATAGACGCAGCAAAGGGAATGTGGCTTTTTTATGAGTACCCTGACACAAAATTTGAAGACTTAAGGTTAA
AGTTCATGGACATAAGAAAAAGAACTTATAGATTTCCTGAACTCGGCAAAAAAGCTTTATTTATCGCAAT
ACCAACCACCAGCGGTACAGGTTCTGAAGTCACAGCCTTTGCAGTTATAACAGACAAAAAGAAAAATATA
AAGTACCCTCTTACAGATTACGAATTGACACCAGATATTGCTATAATAGACCCTGACTTTGTCATGACAA
TTCCACCCTCTGTAACTGCAGATACAGGTATGGATGCTTTAACTCATGCCATTGAAGCTTATGTATCCGT
GATGGCATCAGACTATACAGACGCCCTCGCAGAAAAAGCCATAAAACTGATATTTGAATATCTCCCTAAA
GCATATAAAAACGGACAAGACAAAGTCGCCCGCGAAAAGATGCACAATGCTTCTTGCATAGCAGGAATGG
CATTCACAAATGCATTTTTAGGAATAAACCACAGTATGGCCCACATACTTGGCGCTAAATTCCACCTTCC
CCATGGAAGGGCAAATGCGATACTTCTGCCCTATGTCATAGAATACAATGCTGAGTTACCTAAAAAATTT
GCCTCATTCCCACAATATGAATATCCCAAAGCAGCCGAGAAATACGCAGAAATTGCAAAATTCTTAGGAC
TTCCTGCATCAACCACTGATGAAGGAGTAAAAAGTCTCATTGAAGCAATAAAAAATCTCATGAAAGAGTT
AAATCTTCCTCTTACACTTAAAGATGCGGGAATAAACAAAGAAGAATTTGAAAAACAAATAATGGAAATG
GCTGATATAGCCTTCAACGACCAGTGCACCGGTTCAAATCCAAGAATGCCATTGGTATCAGAAATTGCAG
AAATATATAGAAAAGCCTATGGAGAATAA
[Ka 10]
   SEQ ID NO: 10(*Thermoanaerobacter mathranii*) 
MPTLLQEKKETKEKTEVKETTDVKKQIDLLVERAKRAQEKFMSYTQEQIDEIVKVMALAG
IEKHVELAKLAHEETKMGVYEDKITKNLFAVEYVYNYIKDKKTVGILSENLEENHMEVAE
PVGIIAGVTPVTNPTSTTMFKCLIAIKTRNPIIFSFHPKALKCSIEAAKTMYEAALKAGA
PEGCIGWIETPSIEATQLLMTHPGVSLILATGGAGMVKAAYSSGKPALGVGPGNVPCYIE
KTANIKRAVSDLILSKTFDNGTVCASEQAVIIDEEIANEVKKLMREYGCYFLNKEETKKL
EEFAIDKNTGLMNPAVVGQPAVKIAQMAGFKVPENTKILVAEYPAVGPKYPLSREKLSPI
LALYTVKDYKEGIKRCEEMIQFGGLGHSAVIHSENQQIINEFANRVQASRILVNAPASQG
AIGDIYNTATPSLTLGCGTMGGNSTTDNVSVYNLINIKRVFIRKERMKWFRVPPQIYFER
GSIQYLSQVKGKKAFIVTDPAMVKLGFVDKVTYQLDKANIKYEIFSEVEPDPSVDTVEKG
IKIMKEFEPDLLIAVGGGSAIDAAKGMWLFYEYPDTKFEDLRLKFMDIRKRTYRFPELGK
KALFIAIPTTSGTGSEVTAFAVITDKKKNIKYPLTDYELTPDIAIIDPDFVMTIPPSVTA
DTGMDALTHAIEAYVSVMASDYTDALAEKAIKLIFEYLPKAYKNGQDKVAREKMHNASCI
AGMAFTNAFLGINHSMAHILGAKFHLPHGRANAILLPYVIEYNAELPKKFASFPQYEYPK
AAEKYAEIAKFLGLPASTTDEGVKSLIEAIKNLMKELNLPLTLKDAGINKEEFEKQIMEM
ADIAFNDQCTGSNPRMPLVSEIAEIYRKAYGE
[Ka 11]
   SEQ ID NO: 11(*Thermoanaerobacter ethanolicus*) 
ATGCCTAACTTATTACAAGAAAGAAGAGAAGTAAAAGAAAAAACAGAAGTGAAAGAAACGCTGGATGTTAAAAGACAAAT
AGACCAATTAGTAGAAAAAGCACAAAGAGCACAAGAAAAATTCATGTCTTATACCCAAGAACAAATAGATAAAATAGTTA
AAGCTATGGCTTTGGCTGGTATTGAAAATCATGTGAGATTGGCAAAATTAGCCCATGAAGAAACAAAAATGGGTGTTTAT
GAAGACAAAATAACGAAAAATCTTTTTGCAGTAGAATATGTGTACAATTATATAAAAGACAAAAAGACTGTCGGCGTCCT
TAGTGAAAACTTAGAAGAAAATTATATGGAAGTGGCAGAACCTGTAGGAGTTATAGCCGGCGTAACACCCGTTACAAACC
CTACTTCTACAACAATGTTCAAAAGCTTAATCGCTATTAAAACAAGAAATCCAATAATTTTTAGCTTTCACCCAAAAGCA
TTAAAATGTAGTATAGAAACAGCAAAGACCATGTACGAGGCAGCATTAAAAGCTGGTGCACCAGAAGGATGCATAGGTTG
GATTGAAACGCCTTCAATTGAAGCAACGCAACTTCTCATGACACATCCCGGCGTCTCCCTTATCCTCGCAACAGGCGGCG
CTGGAATGGTAAAAGCCGCTTACAGCTCAGGAAAACCCGCCCTTGGCGTAGGTCCTGGCAATGTCCCCTGCTACATTGAA
AAAACTGCAAATATTAAAAGAGCAGTCTCAGACCTAATACTTAGCAAAACTTTTGACAATGGCACAGTATGCGCATCAGA
ACAAGCTGTGATAATAGACGAAGAAATAGCAGATGAAGTGAAAAAAAATATGAAAGAATATGGTTGCTATTTTCTCAATA
AAGAAGAGACTAAAATGCTAGAAGAATTCGCTATAGACAAAAACACCGGTTTAATGAATCCAGCAGTTGTAGGTCAACCG
GCAACAAAAATTGCTGAAATGGCTGGATTTAAAGTACCTGAAAACACAAAAATACTTGTAGCTGAATATCCTGCTGTAGG
ACCAGAATATCCTCTCTCCAGAGAAAAGCTAAGTCCAATACTTGCTTTATACACAGTCAAAGATTACAACGAAGGCATAA
AAAGATGTGAAGAAATGACAGAATTCGGCGGACTTGGTCACTCTGCAGTAATACATTCTGAAAACCAACAAATTATTAAT
GAATTTGCAAAAAGAGTACGAGCAGGAAGAATTCTTGTAAACGCCCCTGCTTCTCAAGGAGCTATTGGAGATATTTATAA
CACGGCTATCCCATCTTTGACTTTGGGTTGCGGTACAATGGGTAGAAATTCAACAACAGATAATGTAAGTGTTTATAACC
TTATAAACATAAAACGAGTTTTTATAAGAAAAGAGAGAATGAAATGGTTCAGAGTTCCACCACAAATTTATTTTGAAAGA
GGGTCATTACAATATCTGTCACAAGTAAAAGGCAAAAAAGCCTTTATTGTAACAGACCCTGTGATGGTTAAATTAGGTTT
TGTAGATAAAGTAACGTATCAATTAGATAAGGCAAATATTAAATATGAAATATTCTCTGAAGTTGAACCAGATCCTTCAG
TTGACACTGTAGAAAAAGGGGTAAAAATAATGAGAGGGTTTGAGCCTGACTTACTAATAGCTGTAGGTGGTGGTTCTGCA
ATAGATGCAGCGAAGGGAATGTGGCTTTTCTACGAATACCCTGATACAAAATTCGAAGACTTAAGGTTAAAATTTATGGA
CATAAGAAAAAGAACTTATAGATTTCCTGAACTGGGGAAAAAAGCTTTATTTATCGCAATACCAACCACCAGCGGTACAG
GTTCTGAAGTTACTGCCTTTGCAGTTATAACAGACAAAAAGAAAAATATAAAGTACCCTCTCACAGATTACGAATTAACA
CCAGATATCGCTATAATAGACCCTGACCTCACAATGACAATACCACCCTCTGTCACAGCAGACACTGGTATGGACGCTTT
AACCCATGCTATTGAAGCCTATGTATCTGTAATGGCATCAGACTATACAGACGCTCTCGCAGAAAAAGCTATAAAATTGA
TATTTGAATATCTACCTAAAGCATACAAAAACGGGCAAGATAAAGTCGCCCGCGAAAAGATGCACAATGCTTCATGCATA
GCTGGAATGGCCTTCACAAATGCATTTTTAGGAATAAACCATAGTATGGCTCATATACTTGGTGCTAAATTCCATCTTCC
ACACGGAAGAGCAAATGCAATACTTCTGCCCTATGTCATAGAATACAATGCTGAGTTACCTAAAAAATTTGCTTCATTCC
CACAATATGAATATCCCAAAGCAGCCGAGAAATATGCAGAAATTGCAAAATTCTTGGGACTTCCTGCATCAACCATTGAA
GAAGGAGTAAAAAGCCTCATTGAAGCAATAAAAAATCTTATGAAAGAGCTTAATCTTCCTTTGACACTTAAAGAAGCAGG
CATAAATAAAGAAGAATTCGAAAAACAAATAATGGAAATGTCTGATATAGCCTTTAATGACCAGTGCACCGGTTCAAACC
CAAGAATGCCATTGGTATCCGAAATTGCAGAAATATATAGAAAAGCCTATGGAGAATAA
[Ka 12]
   SEQ ID NO: 12(*Thermoanaerobacter ethanolicus*) 
MPNLLQERREVKEKTEVKETLDVKRQIDQLVEKAQRAQEKFMSYTQEQIDKIVKAMALAG
IENHVRLAKLAHEETKMGVYEDKITKNLFAVEYVYNYIKDKKTVGVLSENLEENYMEVAE
PVGVIAGVTPVTNPTSTTMFKSLIAIKTRNPIIFSFHPKALKCSIETAKTMYEAALKAGA
PEGCIGWIETPSIEATQLLMTHPGVSLILATGGAGMVKAAYSSGKPALGVGPGNVPCYIE
KTANIKRAVSDLILSKTFDNGTVCASEQAVIIDEEIADEVKKNMKEYGCYFLNKEETKML
EEFAIDKNTGLMNPAVVGQPATKIAEMAGFKVPENTKILVAEYPAVGPEYPLSREKLSPI
LALYTVKDYNEGIKRCEEMTEFGGLGHSAVIHSENQQIINEFAKRVRAGRILVNAPASQG
AIGDIYNTAIPSLTLGCGTMGRNSTTDNVSVYNLINIKRVFIRKERMKWFRVPPQIYFER
GSLQYLSQVKGKKAFIVTDPVMVKLGFVDKVTYQLDKANIKYEIFSEVEPDPSVDTVEKG
VKIMRGFEPDLLIAVGGGSAIDAAKGMWLFYEYPDTKFEDLRLKFMDIRKRTYRFPELGK
KALFIAIPTTSGTGSEVTAFAVITDKKKNIKYPLTDYELTPDIAIIDPDLTMTIPPSVTA
DTGMDALTHAIEAYVSVMASDYTDALAEKAIKLIFEYLPKAYKNGQDKVAREKMHNASCI
AGMAFTNAFLGINHSMAHILGAKFHLPHGRANAILLPYVIEYNAELPKKFASFPQYEYPK
AAEKYAEIAKFLGLPASTIEEGVKSLIEAIKNLMKELNLPLTLKEAGINKEEFEKQIMEM
SDIAFNDQCTGSNPRMPLVSEIAEIYRKAYGE
[Ka 13]
   SEQ ID NO: 13(*Bacillus coagulans*) 
ATGGCAATCGATGAAAAAGTTGTTGGCAAATCTGCGCAAATCACGGAAATGGTCGATTCC
CTCGTCGCAAAAGGGCAAAAAGCACTGCGTGAATTTATGGAACTGGATCAGGCACAAGTG
GACAATATCGTAAAACAAATGGCACTTGCCGGGTTGGAACAGCATATGGTGCTCGCCAAA
ATGGCCGTGGAAGAAACAGGGCGCGGCGTATATGAAGACAAAATGACAAAAAACTTGTTT
GCAACAGAATACATCTATCACAACATTAAATATAACAAAACAGTCGGCATCATTGACGAA
AACAATAATGAAGGGATTGTTAAATTTGCGGAACCGGTCGGCGTGATTGCCGGGGTCACT
CCTGTAACAAACCCGACATCAACCACGATGTTTAAAGCATTAATTGCCATTAAAACACGC
AACCCAATTATTTTCGCGTTCCATCCGTCCGCACAAAAATGCAGCAGCCATGCCGCAAAA
GTGATGCTTGACGCTGCTGTGAAAGCCGGCGCCCCGGAAAACTGCATCCAATGGATCGAA
AAACCGGCCATTGAAGCGACACAGCAATTGATGAACCATTCGGGTATTTCACTGATTCTT
GCAACGGGCGGTTCCGGCATGGTAAAGTCCGCATATTCGAGCGGGAAACCTGCGCTTGGC
GTCGGGCCGGGAAACGTCCCTTGCTATATTGAAAAATCTGCTGATATCAAACGCGCCGTC
AATGATTTGATTTTGTCCAAAACGTTTGACAACGGCATGATCTGTGCGTCGGAACAGGCT
GTCATCATCGACAAAGAAATTTATGACAATGTGAAAAACGAATTGATTGCAAATCAATGC
TACTTCCTGAATGAAGCGGAGAAGAAAAAGGTTGAGAAAACCGTCATCAATGAAAAGACG
CAATCCGTCAACAGCGCCATTGTCGGCAAACCGGCCTATGAAATTGCGAAAATGGCAGGC
GTAAATGTTCCGGAAGATACCAAAATTTTAATCGCCGAACTTACAGGTGTCGGCCCGGAC
TACCCGCTTTCCAGAGAAAAATTAAGCCCGGTTTTGGCCTGTTACAAAGCCAACAGCACA
AAACAAGGTTTCGAATTTGCAGAAGCCATGCTCGAATTCGGCGGACTTGGTCACTCGGCT
GTGATCCATTCGACAAACGATGAAGTCATTGAACAATACGGCTTAAAAATGAAAGCCGGC
CGGATCATTGTCAACTCCCCGTCTTCGCAAGGGGCAATCGGCGACATTTACAATGCCTAC
ATGCCTTCTTTGACACTCGGCTGCGGCACGTTTGGCGGCAACTCGGTGTCCACAAACGTC
GGTGTCATCAATCTTTACAATGTAAAAACAATGGCGAAACGGAGAGTCAATATGCAATGG
TTTAAAATTCCACCGCGCGTGTATTTTGAAAAGAACTCGGTCCAATACCTGGAAAAAATG
CCGGATATCTCAAGTGCCTTTATTGTCACCGATCCGGATATGGTCAGGCTTGGATTCGTT
GAGAAAGTACTGTATTACTTGCGGAAACGCCCGGACTATGTGCACTGTGAAATTTTCTCA
GAAGTCGAACCGGATCCGTCTATTGAAACGGTTCGGAAAGGTGCCGCAGCTATGGCAAGC
TTCCAGCCGGATGTCATTATCGCGCTTGGCGGCGGGTCGGCGATGGATGCTGCAAAAGGC
ATGTGGCTGTTCTATGAGCATCCGGAAGTTGATTTCAACGAATTGAAGCAAAAATTTATG
GACATCCGGAAACGGGTTGCCAAATTCCCGAAACTGGGTGAAAAAGCGCAGCTGGTCTGC
ATTCCGACCACATCGGGCACCGGTTCTGAAGTGACTTCTTTCGCCGTCATTTCGGATAAA
AAGAACAACACAAAATATCCGCTCGCAGACTATGAACTGACACCGGATGTTGCGATCATT
GACCCGCAGTTTGTTATGACCGTTCCGAAATCCGTAACAGCCGATACCGGGATGGACGTT
TTGACCCATGCGATTGAAGCCTATGTATCCAACATGGCCAACGACTATACGGACGGCCTG
GCAATTAAGGCCATCCAGCTTGTTCACGAATACTTGCCGAAAGCATACGCAAATGGCAAT
GATGCACTGGCACGCGAAAAAATGCACAATGCATCGACTTTGGCAGGGATGGCGTTCTCG
AATGCATTCCTTGGCATCAACCATTCACTTGCCCATAAAATCGGCGCTGAATTCCATATT
CCGCACGGCCGGGCTAACGCGATTCTGTTGCCGCATGTCATCCGCTATAACGCGCAAAAG
CCGAAAAAATTTGCGGCATTCCCGAAATACGAACATTTTATTGCGGATCAACGCTACGCG
GAAATCGCGCGCGTACTTGGTCTTCCGGCAAGCACGACAGAAGAAGGTGTCGAAAGCCTT
TGCCAGGAAATCATCCGGATGTGCAAACTGTTCAATATTCCGCTTAGCCTAAAAGCAGCC
GGCGTGAACCGGGCGGACTTTGAAAAACGGGTTGCCATTATTGCAGACCGCGCATTTGAA
GACCAATGCACACCTGCAAACCCTAAATTGCCGCTTGTCAGCGAATTGGAAGACATTTTA
CGCAAAGCTTTTGAAGGCGTTGAAGCAAAATAA
[Ka 14]
   SEQ ID NO: 14(*Bacillus coagulans*) 
MAIDEKVVGKSAQITEMVDSLVAKGQKALREFMELDQAQVDNIVKQMALAGLEQHMVLAK
MAVEETGRGVYEDKMTKNLFATEYIYHNIKYNKTVGIIDENNNEGIVKFAEPVGVIAGVT
PVTNPTSTTMFKALIAIKTRNPIIFAFHPSAQKCSSHAAKVMLDAAVKAGAPENCIQWIE
KPAIEATQQLMNHSGISLILATGGSGMVKSAYSSGKPALGVGPGNVPCYIEKSADIKRAV
NDLILSKTFDNGMICASEQAVIIDKEIYDNVKNELIANQCYFLNEAEKKKVEKTVINEKT
QSVNSAIVGKPAYEIAKMAGVNVPEDTKILIAELTGVGPDYPLSREKLSPVLACYKANST
KQGFEFAEAMLEFGGLGHSAVIHSTNDEVIEQYGLKMKAGRIIVNSPSSQGAIGDIYNAY
MPSLTLGCGTFGGNSVSTNVGVINLYNVKTMAKRRVNMQWFKIPPRVYFEKNSVQYLEKM
PDISSAFIVTDPDMVRLGFVEKVLYYLRKRPDYVHCEIFSEVEPDPSIETVRKGAAAMAS
FQPDVIIALGGGSAMDAAKGMWLFYEHPEVDFNELKQKFMDIRKRVAKFPKLGEKAQLVC
IPTTSGTGSEVTSFAVISDKKNNTKYPLADYELTPDVAIIDPQFVMTVPKSVTADTGMDV
LTHAIEAYVSNMANDYTDGLAIKAIQLVHEYLPKAYANGNDALAREKMHNASTLAGMAFS
NAFLGINHSLAHKIGAEFHIPHGRANAILLPHVIRYNAQKPKKFAAFPKYEHFIADQRYA
EIARVLGLPASTTEEGVESLCQEIIRMCKLFNIPLSLKAAGVNRADFEKRVAIIADRAFE
DQCTPANPKLPLVSELEDILRKAFEGVEAK
[Ka 15]
   SEQ ID NO: 15(*Geobacillus thermoglucosidasius*) 
ATGGCTGTGGAGGAAAGAGTCGTCGATAAAAAAATCGAAGCAGCAAAAATGATTGATGAG
CTTGTCGCTAATGCACAGAAAGCGTTGGAACAATTTCGCGCTTACGATCAAGAAACGATC
GATCATATCGTGAAAGAAATGGCGTTGGCCGGGCTCGACAAGCATATGGCATTAGCCAAG
CTTGCAGTAGAAGAAACAAAACGTGGTGTATATGAAGATAAAATCATAAAAAACCTTTTT
GCGACAGAATATATATACCACAATATTAAGTACGATAAAACAGTCGGGATTATTCATGAA
AATCCGCATGAAGAAATTATCGAAATTGCTGAGCCTGTTGGTGTTATTGCTGGGATCACG
CCAGTGACAAACCCGACATCGACGACGATGTTTAAAGCGTTAATCTCGATAAAAACGCGC
AATCCGATTATTTTCGCTTTCCATCCATCGGCACAACGATGCAGCAGCGAAGCGGCAAGA
GTGCTGCGCGATGCGGCGGTCCGGGCAGGGGCTCCAGAACATTGCATTCAATGGATTGAA
ACTCCTTCGCTTGATGCAACCAATCAGCTTATGCGCCATCCTGGCGTTTCTCTCATTTTG
GCAACTGGTGGCGCCGGCATGGTGAAAGCAGCGTACAGCTCTGGAAAACCAGCTTTGGGC
GTCGGACCTGGAAATGTGCCTTGCTATATTGAAAAAACGGCAAACATAAAACGGGCGGTA
AATGATTTAATTTTATCGAAAACGTTTGATAACGGCATGATTTGCGCTTCTGAACAAGCA
GTCATTATTGATAAAGAAATTTATGAACAAGTAAAGAAAGAAATGATGGAAAACCATTGT
TATTTCTTAAATGAAGAAGAAAAGAAAAAAGTAGAAAAACTCGTTATCAATGAAAATACA
TGCGCCGTCAACCCGGATATCGTCGGAAAGCCAGCTTATGAAATTGCGAAAATGGCCGGC
ATCGCTGTGCCGGAAGACACAAAAATTCTTGTTGCTGAGCTAAAAGGGGTCGGGCCAAAA
TATCCGTTGTCTCGCGAAAAATTAAGCCCTGTCCTTGCTTGCTATAAAGTTAACAGCACG
GAAGAAGGATTTAAGCGCTGTGAAGAAATGCTGGAATTTGGCGGCTTGGGACATTCGGCT
GTCATCCATTCCGATAATCAAAACGTGGTTACCGAATTTGGCAAACGGATGAAAGCGGGA
CGGATTATCGTTAATGCGCCATCTTCGCAAGGAGCAATCGGCGATATTTACAATGCGTAC
ATTCCGTCATTAACGCTGGGATGCGGCACATTTGGCGGAAACTCTGTTTCGACAAACGTC
AGTGCGATTCATCTTATCAATATAAAAAGAATGGCAAAAAGGACGGTAAATATGCAATGG
TTTAAAGTGCCGCCGAAAATTTATTTCGAAAAAAATGCTGTACAATACTTAGCGAAAATG
CCGGATATTTCCAGAGCCTTTATCGTCACCGACCCGGGAATGGTCAAGCTCGGATATGTC
GATAAAGTGCTGTATTACTTGCGCAGACGCCCGGATTATGTGCATAGTGAAATTTTCTCC
GAAGTAGAGCCAGATCCTTCAATTGAGACGGTAATGAAAGGCGTCGACATGATGAGAAGT
TTCGAGCCGGATGTGATTATCGCGCTCGGAGGCGGCTCGCCAATGGATGCGGCAAAAGCG
ATGTGGCTCTTTTACGAGCATCCGACAGCGGATTTCAACGCATTAAAACAAAAATTTTTA
GATATTCGAAAACGCGTTTATAAATATCCAAAACTGGGCCAAAAAGCGAAATTTGTCGCC
ATTCCGACGACATCAGGAACAGGATCGGAAGTAACGTCCTTTGCCGTCATTACCGATAAA
AAAACGAATATAAAATATCCGTTGGCAGATTATGAATTGACACCGGACGTCGCGATTGTG
GATCCGCAATTTGTCATGACAGTGCCAAAACATGTCACCGCCGATACGGGAATGGATGTA
TTGACACATGCGATCGAAGCGTATGTCTCCAATATGGCCAATGATTATACCGATGGTCTT
GCCATGAAAGCAATCCAACTCGTATTTGAATATTTGCCGCGGGCATATCAAAACGGAGCG
GATGAGCTTGCCCGGGAGAAAATGCATAACGCCTCTACGATTGCGGGAATGGCATTTGCC
AACGCGTTTTTAGGCATTAACCATAGTTTGGCTCATAAACTTGGCGCGGAATTCCATATT
CCGCATGGGCGCGCGAATACCATTTTGATGCCGCATGTCATTCGCTATAACGCAGCGAAA
CCGAAAAAATTTACCGCATTTCCGAAATACGAATATTTCAAAGCGGACCAGCGCTATGCA
GAAATTGCGAGAATGCTCGGCTTGCCGGCCCGCACAACGGAAGAAGGGGTCGAAAGCCTC
GTTCAGGCGATCATTAAGCTGGCAAAACAGTTGGATATGCCGCTGAGCATTGAAGCATGC
GGCGTCAGCAAACAAGAATTTGAAAGCAAAGTTGAAAAATTAGCCGAATTGGCTTTCGAA
GACCAATGTACTACTGCTAACCCGAAACTCCCGCTTGTTAGCGATTTAGTTCATATTTAT
CGCCAAGCGTTTAAAGGAGTTTAA
[Ka 16]
   SEQ ID NO: 16(*Geobacillus thermoglucosidasius*) 
MAVEERVVDKKIEAAKMIDELVANAQKALEQFRAYDQETIDHIVKEMALAGLDKHMALAK
LAVEETKRGVYEDKIIKNLFATEYIYHNIKYDKTVGIIHENPHEEIIEIAEPVGVIAGIT
PVTNPTSTTMFKALISIKTRNPIIFAFHPSAQRCSSEAARVLRDAAVRAGAPEHCIQWIE
TPSLDATNQLMRHPGVSLILATGGAGMVKAAYSSGKPALGVGPGNVPCYIEKTANIKRAV
NDLILSKTFDNGMICASEQAVIIDKEIYEQVKKEMMENHCYFLNEEEKKKVEKLVINENT
CAVNPDIVGKPAYEIAKMAGIAVPEDTKILVAELKGVGPKYPLSREKLSPVLACYKVNST
EEGFKRCEEMLEFGGLGHSAVIHSDNQNVVTEFGKRMKAGRIIVNAPSSQGAIGDIYNAY
IPSLTLGCGTFGGNSVSTNVSAIHLINIKRMAKRTVNMQWFKVPPKIYFEKNAVQYLAKM
PDISRAFIVTDPGMVKLGYVDKVLYYLRRRPDYVHSEIFSEVEPDPSIETVMKGVDMMRS
FEPDVIIALGGGSPMDAAKAMWLFYEHPTADFNALKQKFLDIRKRVYKYPKLGQKAKFVA
IPTTSGTGSEVTSFAVITDKKTNIKYPLADYELTPDVAIVDPQFVMTVPKHVTADTGMDV
LTHAIEAYVSNMANDYTDGLAMKAIQLVFEYLPRAYQNGADELAREKMHNASTIAGMAFA
NAFLGINHSLAHKLGAEFHIPHGRANTILMPHVIRYNAAKPKKFTAFPKYEYFKADQRYA
EIARMLGLPARTTEEGVESLVQAIIKLAKQLDMPLSIEACGVSKQEFESKVEKLAELAFE
DQCTTANPKLPLVSDLVHIYRQAFKGV
[Ka 17]
   SEQ ID NO: 17(*Fonticella tunisiensis*) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCAATAGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Ka 18]
   SEQ ID NO: 18(*Fonticella tunisiensis*)

MKVTNAEELRLKLEEVRKAQKIFASYTQEQVDEIFRQAAMAANNARIKLAKMAVEETGMG
IVEDKVIKNHFASEYIYNKYKDEKTCGVIDRDESFGIMRVAEPIGVIAAIVPTTNPTSTA
IFKALIALKTRNGVIFSPHPRAKKSTIAAAKIVLDAAVKAGAPEGIIGWIDEPTVELSQI
VMKEADMILATGGPGMVRAAYSSGKPAIGVGAGNTPAIIDETAHIKMAVSSILLSKTFDN
GVICASEQSVIVMEQIYDEVKREFEERGAYFLKGEEIDKVRKIILVNGSINANIVGQPAH
KIAEMAGVKVPETAKVLIGEVESVELDEPFSHEKLSPVLAMYKAKTFDEALQKAARLVEL
GGFGHTSVLYTDQVKSRDRIEKFGATMKTGRTIINMPSSQGAIGDIYNFRLAPSLTLGCG
TWGGNSVSENVGVKHLLNIKSVAERRENMLWFRVPEKIYFKYGCLPVALKELKDMGKKRA
FIVTDKVLYNLGFIDTVTKTLDEIGIEYSVFFDVEPDPTLATARKGAAQMKSLNPDVIIA
VGGGSPMDAAKIMWVLYEHPEVRFEDLAMRFMDIRKRVYQFPKMGEKAMMVSIPTSSGTG
SEVTPFAVITDEKTGIKYPLADYELTPDMAIVDAELMLNMPKGLTAASGIDALTHAIEAY
VSVLASEYTNGLALEAIRLIFKYLPQAYNDGPNNIKAREKMAHAATMAGMAFANAFLGVC
HSMAHKLGAMHHVPHGVANGLLINEVIRFNAVDNPRKQTAFPQYKYPNAKFRYARIADYL
NLGGNTEDEKIELLIKAIDDLKNQVNMPKTISEAGVSREKFYATLDEMCEQAFDDQCTGA
NPRYPLISEIKQMYINAFEGISKETEENNK

### Industrial Applicability

The transformants of the present invention are capable of efficiently producing crotyl alcohol, which is an important raw material for butadiene production. Butadiene is in high demand in the industries of the rubber, resin, and other chemical products. Therefore, the transformant of the present invention contributes efficient industrial production of chemical products, while addressing global warming caused by increase of carbon dioxide.

## Claims

1. A transformant obtained by introducing into a *Hydrogenophilus* bacterium any one of the following genes (1) to (5):
(1) A gene encoding an enzyme having crotonaldehyde dehydrogenase activity and a gene encoding an enzyme having crotyl alcohol dehydrogenase activity;
(2) A gene encoding a multifunctional enzyme having both crotonaldehyde dehydrogenase activity and crotyl alcohol dehydrogenase activity;
(3) A gene encoding an enzyme having crotonaldehyde dehydrogenase activity and a gene encoding a multifunctional enzyme having both crotonaldehyde dehydrogenase activity and crotyl alcohol dehydrogenase activity;
(4) A gene encoding an enzyme having crotyl alcohol dehydrogenase activity and a gene encoding a multifunctional enzyme having both crotonaldehyde dehydrogenase activity and crotyl alcohol dehydrogenase activity;
(5) A gene encoding an enzyme having crotonaldehyde dehydrogenase activity, a gene encoding an enzyme having crotyl alcohol dehydrogenase activity, and a gene encoding a multifunctional enzyme having both crotonaldehyde dehydrogenase activity and crotyl alcohol dehydrogenase activity.

2. The transformant according to claim 1, wherein the gene encoding a multifunctional enzyme having both crotonaldehyde dehydrogenase activity and crotyl alcohol dehydrogenase activity is the following DNA (a), (b), (c), (d), or (e):
(a) A DNA comprising the nucleotide sequence of SEQ ID NO: 1;
(b) A DNA comprising a nucleotide sequence having 90% or more identity with SEQ ID NO: 1 and encoding a polypeptide having both crotonaldehyde dehydrogenase activity and crotyl alcohol dehydrogenase activity;
(c) A DNA encoding a polypeptide comprising the amino acid sequence of SEQ ID NO: 2;
(d) A DNA encoding a polypeptide comprising an amino acid sequence having 90% or more identity with SEQ ID NO: 2 and having both crotonaldehyde dehydrogenase activity and crotyl alcohol dehydrogenase activity;
(e) A DNA encoding a polypeptide comprising an amino acid sequence in which 1 to 80 amino acids are deleted, substituted, inserted, or added in the amino acid sequence of SEQ ID NO: 2, and having both crotonaldehyde dehydrogenase activity and crotyl alcohol dehydrogenase activity.

3. The transformant according to claim 1 or 2, wherein the gene encoding an enzyme having crotyl alcohol dehydrogenase activity is the following DNA (f), (g), (h), (i), or (j): (f) A DNA comprising the nucleotide sequence of SEQ ID NO: 3, 5, 7, 9, 11, 13, 15, or 17;
(g) A DNA comprising a nucleotide sequence having 90% or more identity with SEQ ID NO: 3, 5, 7, 9, 11, 13, 15, or 17, and encoding a polypeptide having crotyl alcohol dehydrogenase activity;
(h) A DNA encoding a polypeptide comprising the amino acid sequence of SEQ ID NO: 4, 6, 8, 10, 12, 14, 16, or 18;
(i) A DNA encoding a polypeptide comprising an amino acid sequence having 90% or more identity with SEQ ID NO: 4, 6, 8, 10, 12, 14, 16, or 18, and having crotyl alcohol dehydrogenase activity;
(j) A DNA encoding a polypeptide comprising an amino acid sequence in which 1 to 30 amino acids are deleted, substituted, inserted, or added in the amino acid sequence of SEQ ID NO: 4, 6, 8, 10, 12, 14, 16, or 18, and having crotyl alcohol dehydrogenase activity.

4. The transformant according to any one of claims 1 to 3, wherein the *Hydrogenophilus* bacterium is *Hydrogenophilus thermoluteolus.*

5. A method for producing crotyl alcohol, comprising culturing the transformant according to any one of claims 1 to 4 using substantially only carbon dioxide as a carbon source.
